# EUROPÄISCHE PATENTSCHRIFT

(11) **EP 1 776 330 B1**
(45) Veröffentlichungstag und Bekanntmachung des Hinweises auf die Patenterteilung: **01.09.2010**
(21) Anmeldenummer: 05760701.2
(22) Anmeldetag: 15.06.2005
(51) Int. Cl.: C07C 49/82

(54) **VERFAHREN ZUR CARBONYLIERUNG VON PHENYLALKYLDERIVATEN MIT KOHLENMONOXID**
METHOD FOR CARBONYLATING PHENYLALKYL DERIVATIVES BY MEANS OF CARBON MONOXIDE
PROCEDE DE CARBONYLATION DE DERIVES PHENYLALKYLES AU MOYEN DE MONOXYDE DE CARBONE

(30) Priorität: 30.06.2004 DE 102004031849
(43) Veröffentlichungstag der Anmeldung: 25.04.2007
(73) Patentinhaber: Sanofi-Aventis Deutschland GmbH, 65929 Frankfurt am Main (DE)
(72) Erfinder: JENDRALLA, Heiner, 65931 Frankfurt (DE); BRAUN, Matthias, 65239 Hochheim (DE); KORB, Gerhard, 63512 Hainburg (DE)
(86) Internationale Anmeldenummer: PCT/EP2005/006414
(87) Internationale Veröffentlichungsnummer: WO 2006/002762

(56) Entgegenhaltungen:
- WO-A-95/00480
- WO-A-02/079134
- US-A- 2 634 294
- F.MINISCI ET AL.,: "SOLVENT AND TEMPERATURE EFFECTS IN THE FREE RADICAL AEROBIC OXIDATION OF ALKYL AND ACYL AROMATICS CATALYSED BY TRANSITION METAL SALTS AND N-HYDROXYPHTHALIMIDE: NEW PROCESSES FOR THE SYNTHESIS OF p-HYDROXYBENZOIC ACID, DIPHENOLS, AND DIENES FOR LIQUID CRYSTALS AND CROSS-LINKEL POLYMERS" ORGANIC PROCESS RESEARCH & DEVELOPMENT, Bd. 8, Nr. 2, 2004, Seiten 163-168, XP009056829 20/01/2004 in der Anmeldung erwähnt
- DATABASE BEILSTEIN INSTITUT ZUR FÖRDERUNG DER CHEMISCHEN WISSENSCHAFTEN, FRANKFURT AM MAIN, DE; XP002353920 Database accession no. 1981278 & J. ORG. CHEM.USSR (ENGL.TRANSL.) EN, Bd. 19, Nr. 4, 1983, Seiten 772-775, & INDIAN J. CHEM. SECT. B, Bd. 32, Nr. 4, 1993, Seiten 468-470,
- R. W. MURRAY ANG HONG GU: "LINEAR FREE ENERGY RELATIONSSHIP STUDIES OF THE DIMETHYLDIOXIRANE C-H BOND INSERTION REACTION" J.ORG.CHEM., Bd. 60, 1995, Seiten 5673-5677, XP002353918
- DATABASE BEILSTEIN INSTITUT ZUR FÖRDERUNG DER CHEMISCHEN WISSENSCHAFTEN, FRANKFURT AM MAIN, DE; XP002208568 Database accession no. 937533 & BULL. SOC. CHIM. FR.;FR, 1961, Seiten 2319-2324,
- GLEN A. RUSSELL ET AL: "THE PHOTOBROMINATION OF BRANCHED-CHAIN HYDROCARBONS; THE DARK REACTION OF BROMINE WITH TERTIARY ALKYL BROMIDES" J. AMER.CHEM. SOC, Bd. 77, Nr. 15, 1955, Seiten 4025-4030, XP002364181
- SCOTT V. TRUKSA ET AL: "BENZYLIC HYDROGEN ATOM ABSTRACTION UTILIZING DIETHYL BROMOMALONATE AS RADICAL SOURCE" J.ORG.CHEM., Bd. 57, Nr. 10, 1992, Seiten 2967-2970, XP002364182

## Beschreibung

Die Erfindung betrifft ein Verfahren zur Herstellung von Phenylalkylcarbonsäurederivaten der Formel I mit Kohlenmonoxid in Gegenwart von Supersäuren. Die Erfindung betrifft ferner Halogen-[4-(1-hydroxy-1-methyl-ethyl)-phenyl]-alkyl-1-on-derivate der Formel X.

Die Verfahrensprodukte sind gesuchte Verbindungen für die Herstellung einer Vielzahl von Folgeprodukten, z.B. für die Herstellung von antiallergisch wirkenden Arzneimitteln wie 4-[4-[4-(Hydroxydiphenylmethyl)-1-piperidinyl]-1-hydroxybutyl]-alpha,alpha-dimethylphenylessigsäure, im folgenden Fexofenadin genannt (US 4 254 129). Zentraler Synthesebaustein bei der Herstellung von Fexofenadin ist 2-[4-(4-Chlor-butanoyl)-phenyl]-2-methyl-propionsäure.

Bekannte Verfahren zur Herstellung von 2-[4-(4-Chlor-butanoyl)-phenyl]-2-methyl-propionsäure (EP0703902, WO95/00482, US4254129, WO97/23213, WO97/22344, WO95/00480, WO93/21156, US4254130, WO2003/000658 haben eine hohe Stufenzahl und führen zu p- und m-Stellungs-isomeren, die anschließend voneinander getrennt werden müssen. WO02/79134 beschreibt die Carbonylierung von kernsubstituierten benzylischen Alkoholen der Formel IX zu den entsprechenden Säuren der Formel I auf dem Wege der Koch-Reaktion. Außerdem müssen bei den bekannten Verfahren die Zwischenstufen oft durch Säulenchromatographie gereinigt werden, wodurch die Synthese großer Substanzmengen im Technikum oder Produktionsbetrieb erschwert wird.

Es wurde nun gefunden, dass sich die genannten Nachteile durch eine kurze, effiziente und isomerenfreie Synthese vermeiden lassen, die auch auf aufwendige Reinigungsschritte wie Säulenchromatographie verzichtet.

Die Aufgabe wird dadurch gelöst, dass die Verbindungen der Formel II mit Kohlenmonoxid in Gegenwart einer Supersäure carbonyliert werden. Auf diese Weise wird die Bildung von Stellungsisomeren verhindert und die Verbindungen der Formel (I) können in nur 2 bis 4 Synthesestufen mit hoher Ausbeute und Reinheit hergestellt werden.

Verfahren zur Gewinnung der Verbindung der Formel I 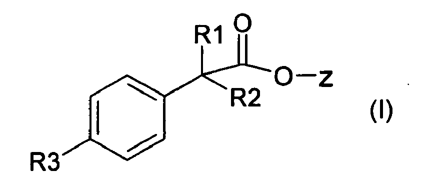 und/oder ein Salz der Verbindung der Formel I, wobei
- R1 und R2: gleich oder verschieden sind und unabhängig voneinander für -(C₁-C₄)-Alkyl stehen,
- R3 für: 1) -C(O)-(C₁-C₄)-Alkyl, worin Alkyl einfach durch Cl oder Br substituiert ist, oder
2) -C(O)-(C₃-C₆)-Cycloalkyl, steht und
- Z für: Wasserstoffatom oder -(C₁-C₁₀)-Alkyl steht,
das dadurch gekennzeichnet ist, dass man eine Verbindung der Formel II, 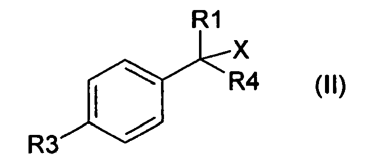 wobei
- R1 und R3: die selben Bedeutungen wie in Formel I haben,
- X für: Cl, Br oder -OH steht und
- R4: die selbe Bedeutung wie der Rest R2 in Formel I hat oder zusammen mit X eine C=C-Doppelbindung darstellt,
mit Kohlenmonoxid oder einer kohlenmonoxid-freisetzenden Verbindung in Gegenwart von konzentrierter Schwefelsäure, Hydrogenfluorid oder einer Supersäure oder Mischungen derselben umsetzt und anschließend
a) Wasser zufügt, um die Verbindung der Formel I zu erhalten, worin Z für Wasserstoffatom steht oder
b) (C₁-C₁₀)-Alkyl-OH zufügt, wenn X für Cl oder Br steht oder R4 zusammen mit X eine C=C-Doppelbindung darstellt, um die Verbindung der Formel I zu erhalten, worin Z für -(C₁-C₁₀)-Alkyl steht.

Die Erfindung betrifft auch ein Verfahren zur Gewinnung der verbindung der Formel I, wobei R1 und R2 gleichzeitig für Methyl stehen,
- R3 für: 1. -C(O)-Propyl, worin Propyl einfach durch Cl substituiert ist, oder
2. -C(O)-Cyclopropyl, steht und
- Z für: Wasserstoffatom oder -(CH₂)ₙ-CH₃, worin n die ganze Zahl Null, 1, 2 oder 3 bedeutet, steht.

Bevorzugt wird im Verfahrensschritt b) Methanol, Ethanol, 1-Propanol oder 1-Butanol zugefügt um die Verbindung der Formel I zu erhalten, worin Z für -(CH₂)ₙ-CH₃, steht und worin n die ganze Zahl Null, 1, 2 oder 3 bedeutet.

Die Erfindung betrifft auch ein Verfahren zur Gewinnung der Verbindung der Formel I, wobei die Umsetzung in Gegenwart von Lösungsmitteln durchgeführt wird, die gegenüber Supersäuren ausreichend inert sind.

Die Erfindung betrifft auch ein Verfahren zur Gewinnung der Verbindung der Formel I, worin Z für Wasserstoffatom steht, das dadurch gekennzeichnet ist, dass man eine Verbindung der Formel I, worin Z für -(C₁-C₁₀)-Alkyl steht, in den entsprechenden Alkohol und Carbonsäure spaltet.

Die Erfindung betrifft auch ein Verfahren zur Gewinnung der Verbindung der Formel I, wobei die Umsetzung in Gegenwart von einem Additiv durchgeführt wird, das in Gegenwart von Kohlenmonoxid in Metallcarbonyle überführt wird.

Unter dem Begriff "(C₁-C₄)-Alkyl" werden Kohlenwasserstoffreste verstanden, deren Kohlenstoffkette geradkettig oder verzweigt ist und 1 bis 4 Kohlenstoffatome enthält, also Methyl, Ethyl, Propyl, Isopropyl, Butyl, Isobutyl, sec-Butyl oder tert.-Butyl.

Unter dem Begriff "-(CH₂)ₙ-CH₃, wobei n die ganze Zahl Null, 1, 2, 3, 4, 5, 6, 7, 8 oder 9 darstellt" oder "-(C₁-C₁₀)-Alkyl" werden Kohlenwasserstoffreste verstanden, deren Kohlenstoffkette geradkettig oder verzweigt ist und 1 bis 10 Kohlenstoffatome enthält, beispielsweise Methyl, Ethyl, n-Propyl, Iso-Propyl, n-Butyl, Iso-Butyl, Pentanyl, Hexanyl, Heptanyl, Octanyl, Nonanyl oder Decannyl.

Für den Fall das n die Zahl Null im Rest "-(CH₂)ₙ-CH₃" darstellt, ergibt sich ein Methylrest. Unter dem Begriff "(C₁-C₁₀)-Alkyl-OH" werden Alkohole verstanden wie Methanol, Ethanol, 1-Propanol, Isopropanol, 1-Butanol, Isobutanol, sec-Butanol, Pentanol, Hexanol, Heptanol, Octanol, Nonanol oder Decanol.

(C₃-C₆)-Cycloalkyl-Reste sind beispielsweise Verbindungen, die sich von 3- bis 6-gliedrigen Monocyclen wie Cyclopropyl, Cyclobutyl, Cyclopentyl, oder Cyclohexyl herleiten.

Unter dem Begriff "kohlenmonoxid-freisetzende Verbindung" werden Verbindungen verstanden, die unter Reaktionsbedingungen Kohlenmonoxid (CO) freisetzen, beispielsweise Ameisensäure, Ameisensäuresalze (Formiate) mit anorganischen oder organischen Kationen, sowie Metallcarbonyle.

Unter dem Begriff "Supersäuren" werden Säuren verstanden, die eine höhere Acidität als konzentrierte Schwefelsäure (Hₒ = -12) aufweisen. Beispiele für Supersäuren sind Protonensäuren wie Perchlorsäure, Chlorsulfonsäure, Fluorsulfonsäure, Trifluormethansulfonsäure, Perfluorbutan-1-sulfonsäure, Lewis-Säuren wie SO₃, Aluminiumtrichlorid oder Antimonpentafluorid oder konjugierte Protonensäure-Lewissäure-Komplexe wie beispielsweise Schwefelsäure mit SO₃ (Oleum; Polyschwefelsäuren), Schwefelsäure mit Borsäure [HB(HSO₄)₄], Fluorsulfonsäure mit Antimonpentafluorid (Magic Acid), Trifluormethansulfonsäure mit Antimonpentafluorid, Hydrogenfluorid mit Antimonpentafluorid (HSbF₆), HF mit TaF₅, BF₃ mit HF (HBF₄, Tetrafluoroborsäure), H₃PO₄ mit BF₃, oder Fluorsulfonsäure mit SO₃, oder konjugierte Komplexe von beispielsweise HSO₃F mit HF und SbF₅, oder HSO₃F mit SO₃ und SbF₅. Solche Supersäuren werden beispielsweise in G.A. Olah, G.K. Surya Prakash, "Superacids", John Wiley & Sons, New York, 1985, Seiten 33-51 beschrieben. Die Definition der Supersäuren wird in G.A. Olah, G.K. Surya Prakash, "Superacids", John Wiley & Sons, New York, 1985, Seiten 4 bis 7 beschrieben; die Definition des Begriffs Supersäure, die hier übernommen wurde, findet sich dort auf Seite 7, die Definition der Säurestärke Hₒ auf Seite 4.

Als inerte Lösungsmittel können beispielsweise flüssiges Schwefeldioxid, superkritisches Kohlendioxid, Sulfolan und n-Alkane mit von 4 bis 12 Kohlenstoffatomen verwendet werden. Bei einigen der Supersäuren können auch Chlorbenzol, Fluorbenzol, Toluol, Cumol, halogenierte Kohlenwasserstoffe, Methylacetat, Ethylacetat, n-Butylacetat und andere Lösungsmittel eingesetzt werden.

Unter dem Begriff "Additiv" werden in erster Linie Verbindungen verstanden, die unter Kohlenmonoxid-Kontakt rasch in Metallcarbonyle übergehen, welche in konzentrierter Schwefelsäure oder Supersäuren einerseits gut löslich sind und andererseits leicht dissoziieren. Beispiele für diese Verbindungen sind Kupfer(I)-oxid, Silber(I)-oxid bzw. Silbernitrat. Als Additive kommen auch preiswerte Metallcarbonyle in Betracht, welche CO-Liganden auf Carbokationen übertragen können, bevorzugt unter anschließender Rückbildung des Ausgangs- Metallcarbonyls durch Reaktion mit CO. Beispiele für derartige Metallcarbonyle sind Eisenpentacarbonyl Fe(CO)₅, Dinatriumtetracarbonylferrat(-2) Na₂Fe(CO)₄, Octacarbonyldicobalt(0) Co₂(CO)₈ und Nickeltetracarbonyl Ni(CO)₄.

Unter dem Begriff "Cyclopropyl-Keto-Derivats von Formel III" versteht man folgende Verbindung:

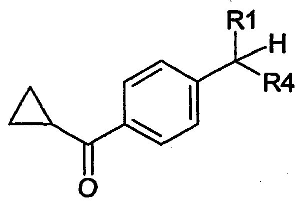

Die genannten Säuren ermöglichen die Carbonylierung des Edukts der Formel (II), indem sie durch Protonierung der Gruppe X, gefolgt von HX-Abspaltung (bzw. im Falle des Alkens, in dem X und R4 zusammen eine α,β-C=C-Doppelbindung darstellen, durch Protonierung dieser Doppelbindung), Carbokationen der Formel (A) erzeugen. Diese reagieren mit Kohlenmonoxid unter Bildung von Acylium-lonen der Formel (B), aus denen durch Reaktion mit Wasser die Carbonsäure der Formel (I) oder durch Reaktion mit einem C₁- bis C₁₀ - Alkohol der entsprechende Ester der Carbonsäure (I) entsteht.

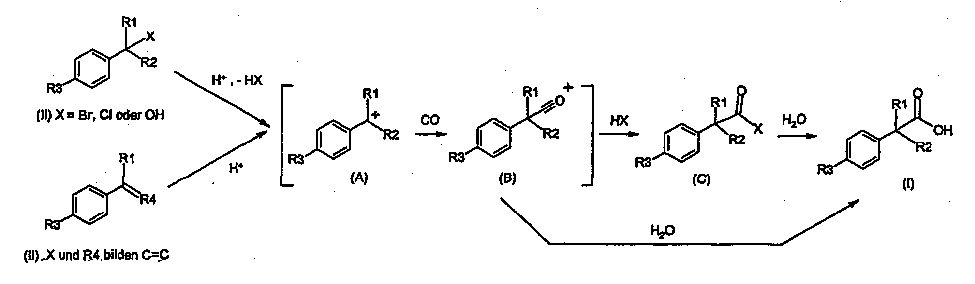

Die Synthese von Carbonsäuren durch Carbonylierung entsprechender Alkohole, Halogenide oder Alkene mit Kohlenmonoxid in Gegenwart starker Säuren ist im Prinzip bekannt. Übersichten zu derartigen Reaktionen findet man in:
H. Bahrmann in "New Syntheses with Carbon Monoxide", E. Falbe Edit., Springer Verlag New York 1980, Kap. 5 "Koch Reactions", Seite 372 - 413;
Houben-Weyl "Methoden der Organischen Chemie", Georg Thieme Verlag Stuttgart 1985, Band E5 (Carbonsäuren und Carbonsäure-Derivate), Seite 315-322;
A.L. Lapidus, S.D. Pirozhkov "Catalytic synthesis of organic compounds by the carboxylation of unsaturated hydrocarbons and alcohols", Russian Chemical Reviews 1989, 58 (2),Seite 117-137;
"Encyclopedia of Reagents for Organic Synthesis", L.A. Paquette Edit., John Wiley New York 1995, Vol. 2, Carbon Monoxide, Reactions with Carbocations, Seite 991.

Diese Reaktion wurde bisher aber noch nicht zur Synthese von Carbonsäuren der Formel (I) aus Edukten der Formel (II) via α,α-Dialkylbenzyl-Carbenium-lonen der Formel (A) eingesetzt. Im Allgemeinen konnten α,α-Dialkylarylessigsäuren bisher nicht per Carbonylierung hergestellt werden, weder durch kationische Carbonylierung, noch durch radikalische oder Übergangsmetall-katalysierte Carbonylierung.

Die Reaktion kann so durchgeführt werden, dass die starke Säure gleichzeitig als Promoter und als Lösungsmittel fungiert. Bei dieser Durchführungsform sind solche Säuren bevorzugt, die billig oder einfach zurückgewinnbar sind und die ein hohes Lösungsvermögen für Kohlenmonoxid haben. Besonders billige Säuren sind beispielsweise Schwefelsäure, Oleum, Schwefelsäure-Borsäure, BF₃-H₃PO₄-Komplex und HF-BF₃, wobei die drei ersteren vorteilhaft aus Umweltgesichtspunkten sind. Trifluormethansulfonsäure oder Perfluorbutan-1-sulfonsäure sind zwar relativ teuer, lassen sich aus wässrigen Aufarbeitungsrückständen aber auf einfache Weise praktisch quantitativ zurückgewinnen und haben wesentlich höhere Lösungsvermögen für Kohlenmonoxid. Gemäß B.L. Booth et al, J.Chem.Soc. Perkin Trans. I, 1979, Seite 2443, lösen sich bei 27 °C und Normaldruck 155 ml CO pro Liter 95% iger Trifluormethansulfonsäure, während sich unter gleichen Bedingungen nur 21 ml CO pro Liter 95%iger Schwefelsäure lösen.

Die 7-fach höhere CO-Konzentration in CF₃SO₃H führt zu höheren Carbonylierungsgeschwindigkeiten des Carbokations (A) und bewirkt, zusammen mit der höheren Säurestärke (Hₒ etwa -14 für CF₃SO₃H, Hₒ etwa -12 für konzentrierte H₂SO₄) verbesserte Ausbeuten der Carbonsäure (I).

Alternativ kann die Reaktion auch in einem Lösungsmittel durchgeführt werden, das bevorzugt gegenüber den genannten starken Säuren inert ist, wobei die starke Säure dann nur als Promoter eingesetzt wird. Als inerte Lösungsmittel können beispielsweise flüssiges Schwefeldioxid, superkritisches Kohlendioxid, Sulfolan und n-Alkane verwendet werden, bei einigen der Supersäuren können beispielsweise auch Chlorbenzol, Fluorbenzol, Toluol, Cumol, halogenierte Kohlenwasserstoffe, Methylacetat, Ethylacetat, n-Butylacetat und andere Lösungsmittel eingesetzt werden.

Alternativ kann die Reaktion bei flüssigen Edukten (II) auch ohne Lösungsmittel durchgeführt werden und bei festen Edukten (II) in Suspension durchgeführt werden. Bei flüssigen Edukten (II) ist dies die bevorzugte Durchführungsform.

In einer weiteren Durchführungsweise führt man die Carbonylierungsreaktion in einem Autoklaven durch, dessen Rührer bevorzugt einen effizienten Eintrag der Gasphase (Kohlenmonoxid) in die Flüssigkeitsphase gewährleistet, zum Beispiel ein Begasungsrührer. Außerdem ist dieser Autoklav mit einer Einrichtung ausgestattet, die ein Zudosieren gegen CO-Druck gestattet. Man legt die Säure und gegebenenfalls ein Additiv, gegebenenfalls gelöst in einem bevorzugt inerten Lösungsmittel, in dem Autoklaven vor. Man verdrängt unter Rühren die Luft im Autoklaven durch Stickstoff und drückt dann Kohlenmonoxid mit dem gewünschten Druck auf. Anschließend dosiert man bei der gewünschten Reaktionstemperatur das Edukt (II), gegebenenfalls gelöst im säure-inerten Lösungsmittel, langsam zu. Man lässt weiter rühren, bis das Zwischenprodukt (B) oder (C) sein Maximum erreicht hat und drückt das Reaktionsgemisch dann, gegebenenfalls nach vorherigem Entspannen der Gasphase, in einen zweiten Reaktor, in dem entweder ein Überschuss Wasser oder ein Überschuss C₁- bis C₁₀-Alkohol nahe 0 °C unter Kühlung vorgelegt wurde. Alternativ kann man Quench / Aufarbeitung des Reaktionsgemischs, zumindest nach Verwendung eines Teils der genannten Supersäuren, auch durch direktes Zudosieren eines Überschusses Wasser oder eines Überschusses C₁- bis C₁₀-Alkohol, unter Kühlung, in den Reaktionsautoklaven durchführen. Diese Aufarbeitungsmethode ist für die Supersäuren ungeeignet, deren Reaktion mit Wasser, oder den genannten Alkoholen zu stark exotherm ist. Beim erwähnten Additiv handelt es um Verbindungen, die unter CO-Kontakt rasch in Metallcarbonyle übergehen, weiche in konzentrierter Schwefelsäure oder Supersäuren einerseits gut löslich sind und andererseits leicht dissoziieren. Auf diese Weise steigern solche Additive die CO-Verfügbarkeit in der flüssigen Phase. Ein bevorzugtes Additiv ist beispielsweise Kupfer(I)-oxid (Cu₂O). Es geht in <80%iger Schwefelsäure bei CO-Kontakt ins Kupfer(I)-monocarbonyl-lon Cu(CO)⁺ über. In >80%iger Schwefelsäure und in Supersäuren erfolgt jedoch weitere CO-Anlagerung unter Bildung des Kupfer(I)-tricarbonyl-lons Cu(CO)₃⁺, wobei das Gleichgewicht durch erhöhten CO-Druck, erniedrigte Temperatur und erhöhte Säurestärke zugunsten von Cu(CO)₃⁺ verschoben wird. In 100%iger Schwefelsäure liegen beispielweise bei -10 °C unter 1 atm CO 2,2 CO-Liganden pro Cu⁺-lon vor, unter 7 atm CO 3,0 CO-Liganden pro Cu⁺-lon [siehe Y.Souma, H. Sano, J. lyoda J.Org.Chem. 1973, 38, 2016-2020]. In Gegenwart von CO-Akzeptoren wie Carbokationen (A) wird CO aus dem Cu(CO)₃⁺-lon, unter Rückbildung von Cu(CO)⁺, auf die Carbokationen unter Bildung von Acylium-lonen (B) übertragen. Das Cu⁺ agiert dabei als "CO-Carrier" von der Gasphase zu den reagierenden Carbokationen in der Lösung. Das langsame Zudosieren des Edukts (II) zu der vorgelegten Lösung von CO in starker Säure (plus gegebenenfalls inertem Lösungsmittel) dient der Minimierung der Stationärkonzentration von Carbokationen (A). Wenn die Konzentration der Carbokationen (A) zu hoch ist, die Reaktion von (A) mit CO zum Acylium-lon (B) relativ langsam erfolgt und die eingesetzte Supersäure nicht stark genug ist, um jegliche Protonenabspaltungen zu unterdrücken, kann ein Teil der Carbokationen (A) durch Protoneneliminierung aus der α-Position ins Alken der Formel (II) übergehen (X und R4 bedeuten zusammen eine C=C-Doppelbindung), das dann mit verbliebenen Carbokationen (A) in Form einer kationischen Polymerisation unter Bildung von Oligomeren und polymeren Produkten reagieren kann. Das Zudosieren des Edukts, der Einsatz geeigneter Additive und die Verwendung von Rührern mit besonders effektivem Gaseintrag in die Flüssigkeitsphase, bewirken signifikante Ausbeuteverbesserungen der gewünschten Carbonsäure (I), wenn:
- die eingesetzte Promoter-Säure nur schlechtes bis mäßiges Lösevermögen für CO besitzt,
- die Säurestärke der Promoter-Säure am unteren Rand der zur Carbokation-Erzeugung notwendigen Säurestärke liegt,
- die Carbonylierung bei besonders niedrigem CO-Druck durchgeführt wird.

Bei Verwendung besonders geeigneter Promoter-Säuren mit hohem CO-Lösevermögen und hoher Säurestärke H_{O} ,wie zum Beispiel Trifluormethansulfonsäure, kann bei CO-Drücken ab etwa 20 bar ohne Ausbeuteminderung auf die Zudosierung des Edukts, die Verwendung von Additiven und speziellen Rührern verzichtet werden. Dies führt zu den beiden folgenden Prozeduren, die technisch besonders einfach durchführbar sind:
A) Man legt die Supersäure, konzentrierte Schwefelsäure oder Hydrogenfluorid, gegebenenfalls gelöst in einem inerten Lösungsmittel, in einem Autoklaven vor. Man verdrängt unter Rühren die Luft im Autoklaven durch Stickstoff und drückt dann Kohlenmonoxid auf. Man lässt etwa 30 Minuten rühren, damit sich die flüssige Phase mit CO sättigt. Dann entspannt man die Gasphase, gibt die Gesamtmenge des Edukts (II) auf einmal unter Luftausschluss zu und drückt sofort wieder CO bis zum gewünschten Druck auf. Quench /Aufarbeitung erfolgen wie oben beschrieben.
B) Man legt in einem Reaktor das Edukt (II), gegebenenfalls.gelöst in einem inerten Lösungsmittel, vor. In einem zweiten Reaktor verdrängt man die gelöste Luft aus der Supersäure und sättigt die Supersäure gegebenenfalls mit Kohlenmonoxid (CO). Man drückt dann die Supersäure auf einmal in den Reaktor mit dem vorgelegten Edukt und drückt sofort anschließend CO bis zum gewünschten Druck auf. Quench / Aufarbeitung erfolgen wie oben beschrieben.

Die Carbonylierungen werden bei einem CO-Druck von 1 bar bis 500 bar, bevorzugt von 1 bis 40 bar, besonders bevorzugt von 5 bis 25 bar, durchgeführt.

Die Reaktionstemperatur bei der Carbonylierung beträgt von -70 °C bis +100 °C, bevorzugt von -10 °C bis +50°C, besonders bevorzugt von 0 °C bis +40 °C.

Die Reaktionszeit liegt im allgemeinen im Bereich von 5 Minuten bis 2 Tagen, vorzugsweise von 15 Minuten bis 5 Stunden, je nach Zusammensetzung des Reaktionsgemischs, gewähltem Temperaturbereich und CO-Druck.

Wenn die Supersäure gleichzeitig als Promoter der Carbonylierungsreaktion und als Lösungsmittel fungiert, werden pro Liter Supersäure von 0,1 Mol bis 5,0 Mol des Edukts (II) eingesetzt, vorzugsweise von 0,3 Mol bis 3,0 Mol, besonders bevorzugt von 0,4 Mol bis 2,0 Mol. Wenn die Supersäure nur als Promoter fungiert, d.h. die Reaktion entweder ohne Lösungsmittel oder unter Verwendung eines bevorzugt säure-inerten Lösungsmittels durchgeführt wird, benötigt man weniger Supersäure. Bevorzugte Supersäuren sind Oleum, Schwefelsäure mit Borsäure, HF mit BF₃, BF₃ x H₃PO₄ - Komplex, Aluminiumtrichlorid, Chlorsulfonsäure, Fluorsulfonsäure, Trifluormethansulfonsäure oder Perfluorbutan-1-sulfonsäure, insbesondere Trifluormethansulfonsäure.

Additive ("CO-Carrier") werden in einer Menge von 5Mol-% bis 100 Mol-% bezogen auf das Edukt (II) eingesetzt, bevorzugt von 10 bis 30 Mol-%, insbesondere etwa 20 Mol-%.

Die Erfindung betrifft auch ein Verfahren, das dadurch gekennzeichnet ist, dass man die Verbindung der Formel II mit Kohlenmonoxid oder einer kohlenmonoxid-freisetzenden Verbindung in Gegenwart von Wasser, wobei Wasser in einer Menge von 2 Mol-% bis 800 Mol-% bezogen auf die Verbindung der Formel II eingesetzt wird, und einer konzentrierten Schwefelsäure oder Hydrogenfluorid oder einer Supersäure oder Mischungen derselben, umsetzt.

Bei der Carbonylierung des Bromids der Formel (II) [X = Bromo, R1 = R2 = Methyl, R3 = 4-Chlorbutyryl] in Trifluormethansulfonsäure hängt die Vollständigkeit des Umsatzes des Edukts (II), sowie die erzielte Ausbeute und Reinheit der gewünschten Carbonsäure (I) vom Wassergehalt der Trifluormethansulfonsäure ab. Es ist vorteilhaft dem Reaktionsgemisch Wasser zuzufügen. Wasser wird in einer Menge von 50 Mol-% bis 500 Mol-% bezogen auf das Edukt (II) zugesetzt, bevorzugt von 90 bis 300 Mol-%, insbesondere etwa 200 Mol-%.

Das Einbringen des Wassers in den Autoklaven kann gemäß unterschiedlichen Arbeitsweisen erfolgen. Das Wasser kann von Anfang an im Autoklaven anwesend sein. Die berechnete Wassermenge kann beispielsweise der Trifluormethansulfonsäure zugesetzt werden. Die Reaktion kann dann wie oben gezeigt durchgeführt werden.

Bei größeren Ansätzen kann es vorteilhaft sein, die benötigte Wassermenge erst während der laufenden Carbonylierung einzubringen. In diesem Fall befüllt man den Autoklaven zuerst mit der trockenen Trifluormethansulfonsäure. Nach dem Inertisieren, Einstellen der Reaktionstemperatur und Einspritzen der Eduktlösung kann dann eine berechnete Menge wasserhaltiger Trifluormethansulfonsäure, beispielsweise Trifluormethansulfonsäure-Monohydrat, zudosiert werden. 100 mol% Wasser werden durch die Umsetzung des Acylium-Ions (B) zur Carbonsäure (I) im Carbonylierungsgemisch verbraucht.

Die direkte Zugabe von Wasser in die Carbonylierungslösung ist sehr exotherm und ist daher möglichst zu vermeiden.

Die Isolierung der Carbonsäure der Formel (I), bzw. dessen Methyl- oder Ethylester, aus der mit einem Überschuss Wasser, oder einem Überschuss des genannten Alkohols versetzten Carbonylierungslösung erfolgt mit Methoden, die dem Fachmann bekannt sind. Die optimale Isolierungsmethode hängt von der An- oder Abwesenheit organischer Lösungsmittel, der Natur der Substituenten R1, R2 und R3, sowie der Natur der verwendeten Supersäure und gegebenenfalls des Additivs ab.

Prinzipien der Isolierung sind:
- Bei Abwesenheit organischer Lösungsmittel direkte Kristallisation der Carbonsäure (I) aus der wässrigen Lösung, gefördert durch Kühlung und Animpfen mit authentischer Carbonsäure.
- Extraktion des sauren wässrigen Gemischs mit einem geeigneten, nicht mit Wasser mischbaren organischen Lösungsmittel, beispielsweise Toluol, Ethylacetat, MTB-Ether oder Dichlormethan, gefolgt vom Waschen der organischen Extrakte mit Wasser zur Entfernung von Resten von Promoter-Säure, gefolgt von Filtration der organischen Phase und Verdampfen des Lösungsmittels, zum Beispiel unter verminderten Druck.

Die Isolierung der Carbonsäure (I) [R1 = R2 = Methyl, R3 = 4-Chlorbutyryl] aus der Carbonylierungsreaktionen in Trifluormethansulfonsäure wird in den Beispielen beschrieben.

Vorteile des erfindungsgemäßen Verfahrens sind beispielsweise bei Verwendung von Trifluormethansulfonsäure, eine saubere und quantitative Reaktion. Die Reinheit bereits der rohen Carbonsäuren (Verbindung der Formel I) betrug gemäß HPLC - Analysen mehr als 95 bis 99 Flächen-Prozent, was auch durch ¹H-NMR-Spektren der rohen, isolierten Carbonsäuren bestätigt wurde.

Die Handhabung aggressiver flüssiger Supersäuren (besonders im großen Maßstab), die Aufarbeitung der Produkte aus der Carbonylierungsreaktion und die Rückgewinnung der Supersäure lassen sich vereinfachen, wenn man letztere in immobilisierter Form auf einem festen Träger einsetzt. Die Herstellung fester Polytrifluormethansulfosiloxan Supersäure auf Trägern wie Kieselgel, Aluminiumoxid oder Bentonite wurde im Stand der Technik schon beschrieben. Diese immobilisierten Katalysatoren wurden erfolgreich für Friedel-Crafts-Acylierungen von Benzolderivaten und Alkylierungen verzweigter Kohlenwasserstoffe eingesetzt (milde Reaktionsbedingungen, bis 98% Ausbeute, mehrfache Wiederverwendung ohne Aktivitätsverlust, kein "Ausbluten" von Trifluormethansulfonsäure in die Reaktionslösung):
- R.-J. Hu, B.-G. Li Catalysis Letters 2004, 98 (1), 43-47 ;
- D.-Q. Zhou, Y.-H. Zhang, M.-Y. Huang, Y.-Y. Jiang Polymers for Advanced Technologies 2003, 14 (3-5), 360 - 363 ;
- F. Boisson, L. Gambut, G. Mignani (Rhodia Chimie) WO 2003080710 A1
- D.-Q. Zhou, C.-M. Wang, J.-H. Yang, M.-Y. Huang, Y.-Y. Jiang Polymers for Advanced Technologies 2002, 13 (3-4), 169 - 172;
- A. de Angelis, C. Flego, P. Ingallina, L. Montanari, M.G. Clerici, C. Carati, C. Perego Catalysis Today 2001, 65 (2-4), 363 - 371;
- D.-Q. Zhou, J.-H. Yang, G.-M. Dong, M.-Y. Huang, Y.-Y. Jiang Journal of Molecular Catalysis A: Chemical 2000,159 (1), 85 - 87;
- F. J.-Y. Chen, C. Le Deore, T. Hamaide, A.M. Guyot, V. Pinjala, J. D.-Y. Ou, US 6060633 A (2000);
- R.L. Mehlberg, G.A. Huff, Jr. (Amoco Corp., USA) WO 9852887 A1 (1998).

Die Erfindung betrifft daher auch die Verwendung von immobilisierter Trifluormethansulfonsäure oder immobilisierter Fluorsulfonsäure für die Carbonylierung von Edukten der Formel (II) zu Produkten der Formel (I).

Als Träger für Trifluormethansulfonsäure (CF₃SO₃H) oder Fluorsulfonsäure (FSO₃H) sind Feststoffe geeignet, bei denen es sich um anorganische Oxide handelt, die freie Hydroxygruppen an ihrer Oberfläche haben. Dabei kann es sich um einfache Oxide wie Siliziumoxid (Kieselgel, Silica), Aluminiumoxid (Alumina), Titanoxid (Titania) oder Magnesiumoxid (Magnesia) handeln, aber auch um mehrfache und komplexe Oxide wie Silica-Alumina, Silica-Alumina-Thoria, Zeolite oder Tonerden (Clays). Beispiele solcher anorganischer Oxide beinhalten Silica, Alumina, Titania, Magnesia, Silica-Alumina, Silica-Titania, Silica-Magnesia, Silica-Alumina-Thoria, Silica-Alumina-Zirconia, kristalline Aluminosilicate einschließlich synthetischer Zeolite wie A-, X- und ZSM-5 Zeolite, natürlich vorkommende Zeolite wie Faujasit und Mordenit, sowie Tonerden wie Bentonit und Montmorillonit.

Trifluormethansulfonsäure oder Fluorsulfonsäure können entweder chemisch an den fester Träger gebunden werden oder nur physikalisch an der Trägeroberfläche stark adsorbiert werden.

Die chemische Bindung an den festen Träger kann mit unterschiedlichen Methoden erfolgen. Zum Beispiel kann man Trifluormethansulfonsäure durch Bildung von Polytrifluormethansiloxanen an der Trägeroberfläche immobilisieren, wie dies beschrieben wurde von:
a) R.-J. Hu, B.-G Li, Catalysis Letters 2004, 98 (1), 43-47
b) D.-Q. Zhou, Y.-H. Zhang, M.-Y. Huang, Y.-Y. Jiang, Polymers for Advanced Technologies 2003, 14 (3-5), 360-363
c) D.-Q. Zhou, J.-H. Yang, G.-M. Dong, M.-Y. Huang, Y.-Y. Jiang, journal of Molecular Catalysis A: Chemical 2000, 159, 85-87

Dabei wird der feste Träger, bevorzugt ein Siliziumoxid sehr geringer Dichte und großer Oberfläche (sogenanntes "fumed silica") oder Aluminiumoxid mit ortho-Ethylsilicat, Wasser, CF₃SO₃H und Ethanol unter Rückfluss gekocht, das Lösungsmittel abdestilliert und der feste Rückstand auf etwa 180 °C erhitzt. Das Prinzip dieser chemischen Bindung ist im folgendem Schema skizziert:

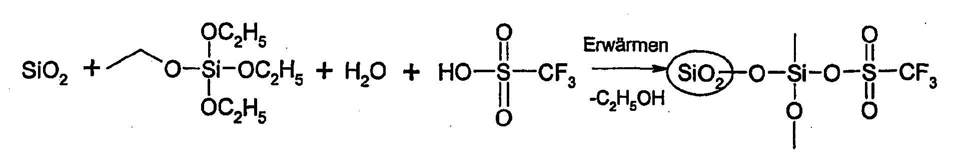

Alternativ kann die chemische Bindung der Supersäure auch erfolgen, indem man den durch Erhitzen im Vakuum dehydratisierten festen Träger in einem inerten Lösungsmittel mit einem Alkylmetallhalogenid XₙMRₘ umsetzt und das erhaltene Produkt mit der Supersäure umsetzt. Dabei bedeutet X ein Halogenid, M bedeutet ein Metall, bevorzugt Aluminium, Bor, Zinn oder Magnesium, und R bedeutet ein monovalentes Kohlenwasserstoff-Radikal. n und m sind ganze Zahlen, die den Valenzanforderungen von M entsprechen. Sowohl n als auch m können null sein. Im ersten Fall liegt eine Alkyl-Metall-Verbindung vor, im letzten Fall ein Metallhalogenid. Bevorzugte Bedeutungen von XₙMRₘ sind AlCl₃, C₂H₅AlCl₂, (C₂H₅)₂AlCl, (C₂H₅)₃Al, BCl₃, SnCl₄ und MgBu₂, wobei Bu für Butylrest steht.

Diese Methode wurde beschrieben von:
d) F. J. Chen, C. LeDeore, T. Hamaide, A.M. Guyot, V. Pinjala, J.D.-Y. Ou, US 6,060,633 (2000)

Das Prinzip dieser chemischen Bindung ist beispielhaft im folgenden Schema skizziert: 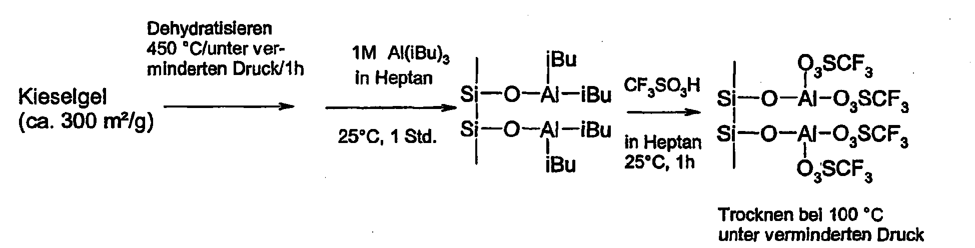 iBu steht für den Rest Isobutyl

Alternativ kann man die Supersäure auch 24 Stunden mit dem festen Träger, bevorzugt Kieselgel 60, unter Feuchtigkeitsausschluss knapp unterhalb des Siedepunkts (bei CF₃S0₃H auf 150 °C) erhitzen.

Die Methode wurde beschrieben von:
e) A. de Angelis, C. Flego, P. Ingallina, L. Montanari, M.G. Clerici, C. Carati, C. Perego, Catalysis Today 2001, 65, 363-371

Unter den Reaktionsbedingungen kommt es eindeutig zu einer Wasserabspaltung und der Immobilisierung von CF₃SO₃H H₂O an der Trägeroberfläche. Es konnte aber nicht eindeutig geklärt werden, ob das Trifluormethansulfonsäure-Hydrat in nur physikalisch gebundener (stark adsorbierter) hoch-dispergierter Form auf der Trägeroberfläche sitzt, oder ob die Trifluormethansulfonsäure mit vicinalen Silanol-Gruppen unter Ausbildung von echten chemischen Bindungen reagiert. Das Prinzip ist im folgenden Schema skizziert:

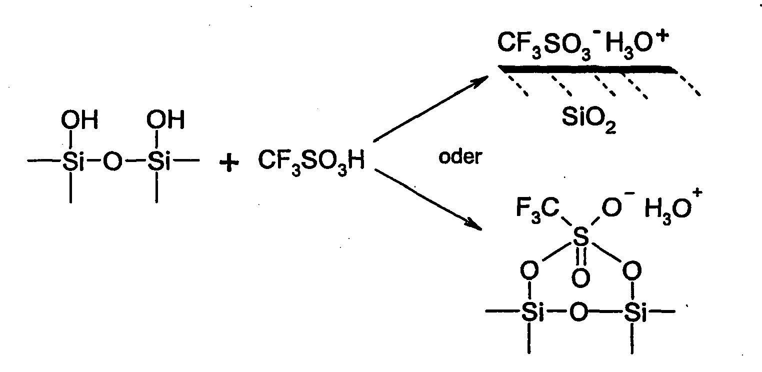

Der feste Träger, bevorzugt Kieselgel, kann auch bei Raumtemperatur mit der Supersäure, bevorzugt Trifluormethansulfonsäure oder Fluorsulfonsäure, imprägniert werden.

In diesem Fall ist die Supersäure nur physikalisch an der Trägeroberfläche gebunden (adsorbiert). Die Adsorption ist aber so fest, dass in einem Strömungsreaktor, der mit einem Kieselgel-Festbett gefüllt ist, die Supersäure bei Aufgabe von der Eingangsseite des Reaktors nur innerhalb einer dünnen Adsorptionszone unmittelbar hinter dem Eingang gebunden ist. Diese Adsorptionszone fungiert dann als katalytische Reaktionszone. Gibt man einen kontinuierlichen Eduktstrom von der Eingangsseite in den Reaktor, so wandert diese katalytische Supersäure-Zone auf dem Festbett nur sehr langsam in Richtung des Eduktstroms, wesentlich langsamer als der Eduktstrom selbst, der in der katalytischen Zone jeweils in den Produktstrom umgewandelt wird. Diese Methode wurde beschrieben von:
f) R. Mehlberg, R.A. Kretchmer (Amoco Corp.) WO 98/52887
g) Hommeltoft et al. (Topsoe Haldor AS) US 5,245,100 h) Hommeltoft et al. (Topsoe Haldor AS) US 5,220,095

Weitere Varianten der Immobilisierung von Supersäuren , insbesondere von Trifluormethansulfonsäure, wurden beschrieben von:
i) E. Benazzi, J.F. Joly (Institut Francais Du Petrole) EP 0 761 306 (1996)
j) F. Chen, A. Guyot, T. Hamaide, C. LeDeore (Exxon) WO 95/26814
k) L.R. Kallenbach, M.M. Johnson (Phillips Petroleum Company) US 5,349,116 (1994)

Die Carbonylierungsedukte der Formel (II) können, wie im folgenden Schema skizziert, in zwei bis drei Stufen aus dem Isoalkylbenzol der Formel (V) hergestellt werden, das entweder kommerziell erhältlich ist (R¹ = R⁴ = CH₃ , sowie R¹ = Ethyl, R⁴ = Methyl) oder einfach durch Friedel-Crafts-Alkylierung von Benzol mit kommerziell verfügbarem Alkylchlorid der Formel (VI) oder alternativ durch säurekatalysierte elektrophile Addition des Alkens der Formel (VII) an Benzol erhalten wird.

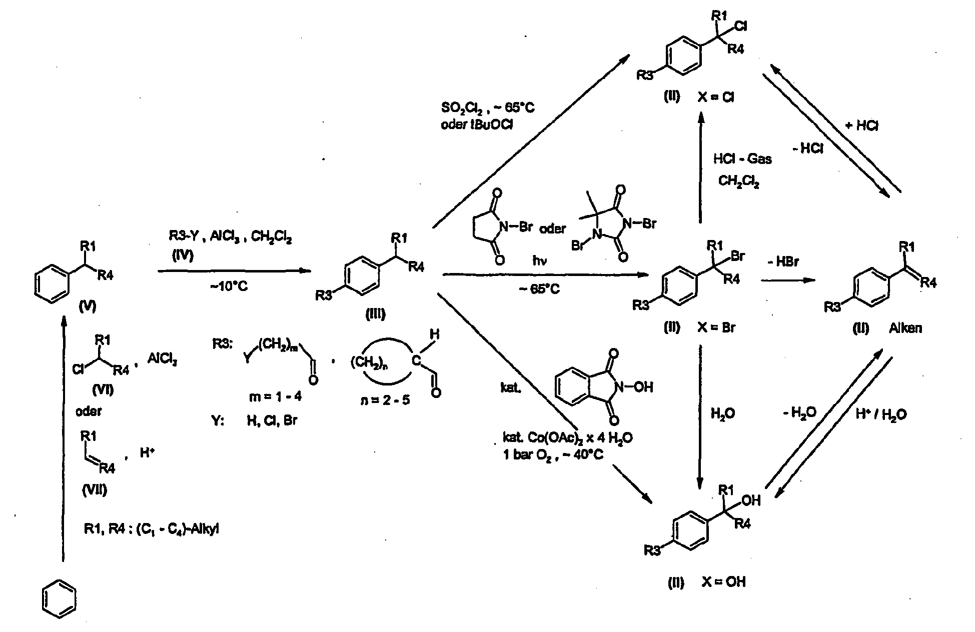

Verbindungen der Formel (III) sind bekannt (WO 95/00480 Anspruch 7, Seite 179). Ein Beispiel für eine Friedel-Crafts-Acylierung eines Isoalkylbenzols der Formel (V) (Cumol) mit einem Acylhalogenid der Formel (IV) (4-Chlorbuttersäurechlorid) findet man in WO 95/00480 (Beispiel 1, Seite 39). Die Reaktion erfolgt in guter Ausbeute mit sehr hoher para-Selektivität. Der Anteil von Positionsisomeren in (III) liegt bei nur 0 bis 0,2 % (siehe auch in Referenzbeispielen 1 und 2).

Bromide der Formel (II) (X = Br) sind bekannt (WO 95/00480, Anspruch 7, Seite 179). Drei Durchführungsformen für die benzylische Bromierung von (III) (R¹ = R² = Methyl, R³ = 4-Chlorbutyryl) sind in WO 95/00480 (Beispiel 4, Seiten 47-49) enthalten. Bei der dortigen "Methode A" (Seite 47) wurde (III) mit 1,05 Äquivalenten N-Bromsuccinimid (NBS) in Tetrachlorkohlenstoff in Gegenwart katalytischer Mengen Dibenzoylperoxid (0,13 mol%) eine Stunde am Rückfluss erhitzt. Bei der dortigen "Methode B" (Seite 48) wurde (III) mit 1,045 Äquivalenten NBS in Tetrachlorkohlenstoff in Gegenwart katalytischer Mengen 2,2'-Azobisisobutyronitril (AIBN, 8,2 mol%) unter Stickstoffatmosphäre auf 80°C erwärmt, bis die exotherme Radikalkettenreaktion einsetzte. Nach 30 Minuten am Rückfluss wurden weitere 0,025 Äquivalente NBS zugegeben und weitere 15 Minuten refluxiert. Bei der dortigen "Methode C" (Seite 48) wurde eine Lösung von (III) in Dichlormethan mit einer wässrigen Lösung von Natriumbromat (NaBrO₃, 0,35 Äquivalente) versetzt und unter Rühren bei 10 °C belichtet. Weitere 0,70 Äquivatente wässrige Natriumbromat-Lösung wurden langsam zugetropft, noch 2 Stunden gerührt und weitere 30 Minuten belichtet.

Ein weiterer Aspekt der Erfindung betrifft Verbindungen der Formel X, 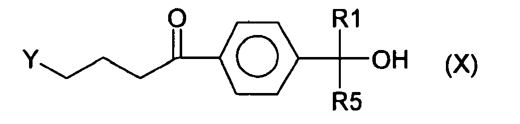 wobei Y Chlor- oder Bromatom, bedeutet, und
R1 und R5 gleich oder verschieden sind und unabhängig voneinander für -(C₁-C₄)-Alkyl stehen.

Die Erfindung betrifft ferner Verbindungen der Formel X, worin
Y ein Chloratom bedeutet und R1 und R5 jeweils für Methyl stehen.

Die Erfindung betrifft ferner ein Verfahren zur Gewinnung von Verbindungen der Formel X, das dadurch gekennzeichnet ist, dass man
a) eine Verbindung der Formel III, 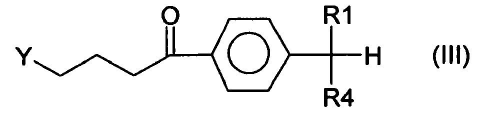 wobei Y Chlor- oder Bromatom bedeutet, und R1 und R4 gleich oder verschieden sind und unabhängig voneinander für -(C₁-C₄)-Alkyl stehen, mit Sauerstoff in Gegenwart von Kobalt(II)acetat-Tetrahydrat und N-Hydroxyphthalimid umsetzt, oder
b) eine Verbindung der Formel XI, 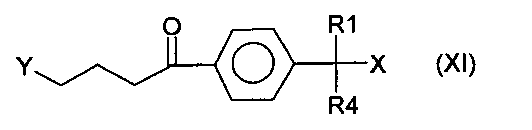
   wobei Y Chlor- oder Bromatom, bedeutet,
   X für Chlor- oder Bromatom steht,
   R1 und R4 gleich oder verschieden sind und unabhängig voneinander für -(C₁-C₄)-Alkyl stehen oder
   R4 und X zusammen eine C=C-Doppelbindung darstellen,
   mit Wasser umsetzt.

Benzylische Alkohole der Formel (II) (X = OH) sind neu. Sie können gemäß dem obigen Schema durch direkte, benzylische radikalische Oxidation von Verbindungen der Formel (III) hergestellt werden, wobei Sauerstoff bei Normaldruck als Oxidationsmittel, Kobalt(II)acetat-Tetrahydrat und N-Hydroxyphthalimid als Katalysatoren verwendet werden (siehe auch Beispiel 27).

Die eng verwandte benzylische Oxidation von Cumol nach gleicher Methode wurde von F. Minisci et al, Proc. Res. & Dev. 2004, 8, 163 - 168 beschrieben.

Alternativ kann man die Alkohole der Formel (II) (X = OH) auch hydrolytisch aus den Bromiden der Formel (II) (X = Br) herstellen (siehe auch Beispiel 28). Analog kann man die Alkohole der Formel (II) (X=OH) auch aus den Chloriden der Formel (II) (X = CI) gewinnen, entweder durch direkte Hydrolyse, oder über das Alken als Zwischenprodukt.

Nachfolgend ist die Erfindung an Hand von Beispielen näher erläutert.

Endprodukte werden in der Regel durch ¹H-NMR (400 MHz, in CDCl₃ oder DMSO-d₆) bestimmt. Temperaturangaben in Grad Celsius, RT bedeutet Raumtemperatur (22 °C bis 26 °C), Min bedeutet Minute. Verwendete Abkürzungen sind entweder erläutert oder entsprechen den üblichen Konventionen. Die Carbonylierungs-Screeningexperimente wurden in einem Reaktorblock durchgeführt, der aus acht 2 ml-Edelstahlautoklaven bestand. Die acht Mini-Autoklaven eines Blocks werden in den folgenden Beispielen mit A1 bis A8 gekennzeichnet. Jeder dieser Mini-Autoklaven war mit einem kreuzförmigen 9 x 9 mm Magnetrührstäbchen ausgestattet. Das Verdrängen der Luft aus der Gas- und Flüssigkeitsphase ("Sekurieren") erfolgte vollautomatisch, gesteuert durch eine Software. Dabei wurde das Reaktionsgas CO dreimal mit jeweils 5 bar aufgedrückt und der Autoklav dann jeweils auf ein schwaches Vakuum evakuiert (auf etwa 0,5 bar). Die präparativen Carbonylierungsexperimente wurden in einem 500 ml Büchl-Autoklaven aus Hasteloy durchgeführt, der einen Begasungsrührer enthielt. Der Begasungsrührer wurde auf eine Drehzahl von 1000 Umdrehungen / Minute eingestellt.

### Referenzbeispiel 1:

### Synthese von 4-Chlor-1-(4-isopropyl-phenyl)-butan-1-on (Formel III, R¹ = R² = Methyl, R³ = 4-Chlorbutyryl) aus Isopropylbenzol (Cumol)

In einem 2 L-Vierhalskolben mit mechanischem Flügelrührer wurden unter N₂-Atmosphäre 166,70 g (1,25 Mol) Aluminiumchlorid und 605,14 g (455 ml) Dichlormethan vorgelegt. Zu dieser Suspension wurde bei einer Innentemperatur von +10°C innerhalb von 30 Minuten 176,33 g (139,9 ml, 1,25 Mol) 4-Chlor-buttersäurechlorid zudosiert. Anschließend wurde innerhalb von 40 Minuten 142,73 g (166 ml, 1,187 Mol) Cumol bei 10°C zugetropft. Dabei entwich HCl-Gas. Bei +10°C wurde 45 Minuten nachgerührt. Bereits nach 5 Minuten war der Umsatz vollständig. Die gelbe Reaktionslösung wurde innerhalb 90 Minuten in 1000 g Eiswasser dosiert. Es wurde bei 0 - +5°C 30 Minuten nachgerührt. Während einer weiteren Nachrührzeit von 90 Minuten erwärmte sich die Mischung auf RT. Die Phasen wurden getrennt. Die wässrige Phase wurde zweimal mit je 532 g (400 ml) Dichlormethan bei RT nachextrahiert. Die vereinigten organischen Phasen wurden bei 20°C einmal mit 412,10 g (400 ml) 5%iger Natriumhydrogencarbonatlösung gewaschen. Die Dichlormethanphase wurde am Rotatationsverdampfer bei 30°C Badtemperatur unter verminderten Druck soweit wie möglich eingeengt. Man erhielt 280,3 g gelbes Öl. Es wurde in 240,60 g (280 ml) Isopropanol / Wasser-Gemisch (2:1) aufgenommen, auf 0°C abgekühlt und 2 Stunden bei 0°C nachgerührt. Schon nach etwa 5 Minuten begann bei 0°C die Kristallisation aus der milchig-trüben Emulsion. Das kristalline Produkt wurde über eine Filternutsche abgesaugt und mit 128,60 g (150 ml) Isopropanol / Wasser-Gemisch (2:1) gewaschen. Der Feststoff wurde unter verminderten Drucktrockenschrank bei RT unter N₂-Überlagerung getrocknet. Man erhielt 253,4 g (1,128 Mol, 95,0 % der Theorie (d. Th.)) feine, farblose, flockenförmige Kristalle, HPLC-Reinheit 98,8%, Schmelzpunkt (Schmp.) 38-39°C. ¹H-NMR (CDCl₃): δ = 1,28 (d, 6H, 2 x CH₃), 2,23 (qui, 2H, CH₂), 2,98 (sept, 1H, CH), 3,16 (t, 2H, CH₂), 3,68 (t, 2H, CH₂), 7,32 (∼d, 2H, arom.-H), 7,91 (~d, 2H, arom.-H). MS (Cl+, Lösungsmittel (Lsm.) Methanol (MeOH): m/z = 227/225 (11% / 33%, M+H⁺), 189 (10%, M+H⁺ - HCl), 162 (21%, M+H⁺ - CH₂CH₂Cl), 147 (100%, M+H⁺ - CH₂CH₂CH₂Cl). IR (KBr): ν = 1678 (C=O), 1600 (C=C von Aryl), 1223 cm⁻¹.

### Referenzbeispiel 2:

### Synthese von 4-Chlor-1-(4-isopropyl-phenyl)-butan-1-on (Formel III, R¹ = R² = Methyl, R³ = 4-Chlorbutyryl) aus Isopropylbenzol (Cumol)

In einem 2 L-Vierhalskolben mit mechanischem Flügelrührer wurden unter N₂-Atmosphäre 138,0 g (1,03 Mol) Aluminiumchlorid und 1000 ml Dichlormethan vorgelegt. Zu dieser Suspension wurde bei einer Innentemperatur von +5°C unter Eiskühlung innerhalb von 10 Minuten 142,4 g (113 ml, 1,25 Mol) 4-Chlor-buttersäurechlorid zudosiert. Zu der fast klaren, gelben Lösung wurde anschließend innerhalb von 35 Minuten 120,2 g (139 ml, 1,00 Mol) Cumol zugetropft. Als nach der Hälfte der Zeit eine zunehmende HCl-Gasentwicklung einsetzte, ersetzte man das Eisbad durch ein Wasserbad, so dass nach 20 Min RT erreicht wurde. Man ließ noch 30 Min nachrühren. Die gelbe Reaktionslösung wurde unter Rühren innerhalb 90 Minuten in 1000 g Eiswasser dosiert. Die organische Phase wurde abgetrennt und die wässrige Phase nochmals mit 2 x 200 ml Dichlormethan extrahiert. Die vereinigten organischen Phasen wurden mit 300 ml 5%iger Natriumhydrogencarbonatlösung gewaschen. Die Dichlormethanphase wurde am Rotatationsverdampfer bei 30°C Badtemperatur unter verminderten Druck soweit wie möglich eingeengt, das gelbe Öl in 200 ml n-Heptan aufgenommen und erneut unter verminderten Druck soweit wie möglich eingeengt. Der Rückstand wurde bei RT in 225 ml n-Heptan unter N₂-Atmosphäre klar gelöst. Die Lösung wurde langsam abgekühlt und angeimpft. Bei etwa 19°C begann die Kristallisation. Bei Erreichen von +2°C war ein dicker Kristallbrei entstanden, der noch 10 Minuten nachgerührt und anschließend über eine leicht vorgekühlte Glasfritte abgesaugt wurde. Es wurde mit eiskalten n-Heptan nachgewaschen, bis die Mutterlauge vollständig aus den Kristallen ausgewaschen war. Es wurde scharf abgesaugt und der Feststoff im HV getrocknet. Man erhielt 182,8 g farblose Kristalle (97,9 Fl.-%ig gemäß GC, Schmp. 38°C) und 37,8 g gelbes Öl aus dem Einengen der Mutterlauge. Kristallisation des Öls aus 40 ml n-Heptan lieferte weitere 12,7 g farblose Kristalle (97,7 Fl.-%ig gemäß GC, Schmp. 38°C). Gesamtausbeute: 195,5 g (870 mmol, 87% d. Th.). Spektren waren identisch mit dem Produkt aus Referenzbeispiel 1.

GC-System (FID): 30m Fused Silica Kapillarsäule HP1, 0,53 mm ID, 1,5 µm Schichtdicke der stationären Phase, Säulenfluss: 8,5 ml He / Min ; Temperaturprogramm startete bei 50°C, 2 Min isotherm, dann mit 20°C / Min auf 275°C; tᵣₑₜ Produkt = 11,3 Min, Isomer 11,0 Min, Cumol 5,3 Min

### Beispiel 1A:

### Synthese von 1-[4-(1-8rom-1-methyl-ethyl)-phenyl]-4-chtor-butan-1-on (Formel VIII, Y = CI) hoher Reinheit mit N-Brom-succinimid (NBS) unter Belichtung

In einem 50ml-Dreihals-Sulfierkolben mit Rührfisch, Thermometer und Rückflusskühler wurden unter N₂ 2,30 g (10,0 mmol) 4-Chlor-1-(4-isopropyl-phenyl)-butan-1-on (97,9% ig, aus Referenzbeispiel 2) und 1,93 g (10,8 mmol) N-Brom-succinimid (99%ig, Firma ABCR) in 33 ml Tetrachlorkohlenstoff (Firma Merck Darmstadt) gelöst / suspendiert. Der Kolben wurde bis etwa halbe Höhe in ein Ölbad eingetaucht, das auf 80°C vorgeheizt war, und von schräg oben mit einer Osram Ultra Vitalux-Lampe bestrahlt, die etwa 10 cm Abstand zur Oberfläche der Suspension hatte. Ab etwa 60°C setzte unter Aufschäumen die deutlich exotherme Radikalkettenreaktion ein. Nach erfolgtem Kettenstart wurde das warme Ölbad sofort abgesenkt. Die Aufrechterhaltung der Reaktionstemperatur von 60 °C bis 75°C erfolgte durch die Reaktionswärme und die von der Lampe abgestrahlte Wärme. Das schwere NBS ging in das leichte Succinimid über, welches als weißer Feststoff an der Oberfläche der Suspension schwamm. Nach 5 Min war das Edukt gemäß GC-Analyse einer Probe bereits zu >99% umgesetzt. Nach 15 Min schaltete man die Lampe aus und ließ das Reaktionsgemisch auf RT abkühlen. Das Succinimid wurde abgesaugt und mit wenig CCl₄ gewaschen. Das Filtrat wurde mit 3 x 11 ml kaltem Wasser gewaschen und unter verminderten Druck eingeengt. Der ölige Rückstand wurde mit einem Kristall des Produkts angeimpft, wobei Kristallisation einsetzte und weiter im HV getrocknet. Man erhielt 2,93 g (9,65 mmol, 96% d. Th.) blassbeige Kristalle, Schmelzpunkt 35,5 bis 36,5 °C. Bei GC-Analyse (System wie in Referenzbeispiel 2) spaltete das Produkt weitestgehend HBr ab und wurde als Alken der Formel II detektiert (tᵣₑₜ 11,6 Min, 94,5 Fl.-%). Die GC-Analyse ist für eine grobe Reinheitsbestimmung geeignet. Bei HPLC-Analyse des Bromids erfolgte weitgehende Solvolyse, überwiegend zum Alkohol (X) (Y = Cl) und zum Alken der Formel II. Ausmaß der Solvolyse und genaue Zusammensetzung der Solvolyseprodukte war von der Zubereitung der HPLC-Probe und von der Standzeit der Lösung vor der Injektion abhängig. Die HPLC-Analyse ist darum selbst für eine grobe Reinheitsbestimmung ungeeignet. Am besten erfolgt die Reinheitsbestimmung per ¹H-NMR (CDCl₃) durch Ausmessen der verstärkten Integrale des Produkts, der =CH₂ - Protonen des Alkens (2 x s, 2 x 1H, δ = 5,21 und 5,49), sowie der CH₂Br - Protonen (2 x d, 2 x 1H, δ = 4,13 und 4,37) und der CH₃-Protonen (s, 3H, δ = 2,34) des Dibromids . So wurde ermittelt, dass das Produkt 97,5 Mol%-ig war, 1,6 mol-% des Dibromids, 0,9 mol-% des Alkens und 0 mol-% des Edukts enthielt. Im ¹³C-NMR wurden keine Verunreinigungen gefunden.
¹H-NMR (CDCl₃): δ = 2,20 (s, 6H, 2 x CH₃), 2,23 (qui, 2H, CH₂), 3,17 (t, 2H, CH₂), 3,68 (t, 2H, CH₂), 7,71 (∼dt, 2H, arom.-H), 7,94 (∼dt, 2H, arom.-H). ¹³C-NMR (CDCl₃): δ = 26,87 (CH₂), 35,33 (2xCH₃), 35,47 (CH₂CO), 44,75 (CH₂Cl), 62,44 (C-Br), 126,25 (2 x arom.-CH), 128,22 (2 x arom.-CH), 135,99 (arom.-C), 151,89 (arom.-C), 198,39 (C=0). IR(Feststoff): v = 1679 (C=0), 1604(C=C von Aryl), 1409, 1227, 1092, 842, 776, 738, 728, 612 cm⁻¹.

### Beispiel 1B:

### Synthese von 1-[4-(1-Brom-1-methyl-ethyl)-phenyl]-4-chlor-butan-1-on (Formel VIII, Y = CI) mit 1,3-Dibrom-5,5-dimethylhydantoin in Chlorbenzol unter Belichtung

In einem 50ml-Dreihals-Sulfierkolben mit Rührfisch, Thermometer und Rückflusskühler wurden unter N₂ 2,30 g (10,0 mmol) 4-Chlor-1-(4-isopropyl-phenyl)-butan-1-on (97,9% ig, aus Referenzbeispiel 2) und 1,54 g (5,3 mmol) 1,3-Dibrom-5,5-dimethylhydantoin (98%ig, Aldrich) in 33 ml Chlorbenzol (Firma Merck Darmstadt) gelöst / suspendiert. Der Kolben wurde bis etwa halbe Höhe in ein Ölbad eingetaucht, das auf 63°C vorgeheizt war, und von schräg oben mit einer Osram Ultra Vitalux-Lampe bestrahlt, die etwa 10 cm Abstand zur Oberfläche der Suspension hatte. Ab etwa 58°C setzte die deutlich exotherme Radikalkettenreaktion ein, welche die Innentemperatur innerhalb von etwa 5 Min langsam weiter bis auf 69°C ansteigen ließ, um anschließend wieder zu sinken. Nach insgesamt etwa 10 minütiger Reaktionsdauer wurde das Ölbad entfernt und die Lampe abgeschaltet. Gemäß GC-Analyse einer Probe war das Edukt quantitativ umgesetzt und das Alken wurde mit 92 Flächenprozent (Fl.-%) angezeigt. Die auf RT abgekühlte, gelbe leicht trübe Lösung wurde mit 3 x 10 ml kaltem Wasser gewaschen, unter verminderten Druck eingeengt und am HV getrocknet. Der ölige Rückstand wurde mit einem Kristall des reinen Produkts angeimpft, wobei Kristallisation einsetzte. Das Produkt wurde weiter am HV getrocknet. Man erhielt 3,04 g (10,0 mmol, 100% d. Th.) blassbeige Kristalle, Schmelzpunkt 35 °C bis 36,5°C. GC-Analyse (System wie in Referenzbeispiel 2) zeigte 95,1 Fl.-% Alken an. Analyse per ¹H-NMR, wie in Beispiel 1 beschrieben, ergab 94 mol%-% des gewünschten Produkts, 3 mol-% des Dibromids und 3 mol-% des Alkens.

### Beispiel 2A:

### Synthese von 1-[4-(1-Brom-1-methyl-ethyl)-phenyl]-4-chlor-butan-1-on (Formel VIII, Y = Cl) in einem Methylenchlorid / Wasser-Zweiphasengemisch unter Belichtung und in situ-Generierung des Broms durch Komproportionierung von Natriumbromat und Bromwasserstoff.

Die Reaktion wurde in einer konzentrischen, zylindrischen 250 ml-4-Hals-Belichtungsapparatur (Glas) mit Magnetkreuz, Intensivkühler, Schlauchpumpe, PT100-Wärmefühler und Stickstoffüberlagerung durchgeführt. Im Zentrum des Zylinders befand sich eine UV-Tauchlampe (TQ150, Original Hanau) die mittels eines Kryostaten (Julabo Typ FP 40, Ethanol /Wasser-Gemisch 50:50) gekühlt wurde.

In der Belichtungsapparatur wurden 5,21 g (33,84 mmol) 98% iges Natriumbromat und 12,5 ml Wasser vorgelegt und unter Rühren gelöst. Die Lösung wurde mit Stickstoff überlagert. Es wurden

22,79 g (99,28 mmol) 4-Chloro-1-(4-isopropyl-phenyl)-butan-1-on (97,9 FI.-%ig) und 110 ml Dichlormethan zugegeben. Die auf 0°C vorgekühlte UV-Lampe wurde eingebaut und angeschaltet. Zusätzlich wurde die Belichtungsapparatur von außen mit einem Trockeneis /Ethanol-Gemisch gekühlt. Nach dem Erreichen einer Innentemperatur von 0°C wurde mit der Dosierung (per Schlauchpumpe) von 19,64 g (116,50 mmol) 48%iger wässriger Bromwasserstofflösung begonnen. Diese wurde in 23 Minuten zudosiert. Dabei erwärmte sich die Reaktionslösung auf 2°C. Die Leitungen der Schlauchpumpe wurden mit 5 ml Wasser nachgespült. Die Lampe wurde nach Beendigung der HBr-Dosierung noch für weitere 15 Min zur Vervollständigung der Reaktion weiterbetrieben. Die Reaktionslösung war trüb und farblos. Sie wurde in einen Scheidetrichter überführt und die Phasen nach einer Phasentrennzeit von 10 Min getrennt. Die untere Phase (DCM) wog 173,3 g, die obere Phase (Wasser) wog 32,4 g.

Die organische Phase wurde mit 2 mal 50 ml Wasser gewaschen. Die organische Phase (163,7 g) wurde am Rotationsverdampfer bei 30°C Wasserbadtemperatur bis zum Endvakuum von 20 mbar eingeengt. Dabei erfolgte auch azeotrope Trocknung. Es resultierten 29,04 g eines klaren, nahezu farblosen Öls, das vollständig durchkristallisierte.

GC-Analyse: 0,5 Flächen% Edukt (tret 14,54 min), 98,5 FI.% Produkt (davon 97,8 FI.% detektiert als Alken,tret 14,87 min und 0,7 FI.% als Bromid, tret 15,45 min), 1,0 FI.% Dibromid (tret 16,00 min).

29,04 g (98,5 FI.% = 28,6 g 100%iges Produkt) entsprechen einer Ausbeute von 94,9% d.Th.. Die Spektren entsprachen den in Beispiel 1A beschriebenen Daten.

### Beispiel 2B:

### Synthese von 1-[4-(1-Brom-1-methyl-ethyl)-phenyl]-4-chlor-butan-1-on (Formel VIII, Y = CI) in einem anfänglichen Dreiphasengemisch (Methylenchlorid / Wasser und ungelöstes, festes Natriumbromat) unter Belichtung und in situ-Generierung des Broms durch Komproportionierung von Natriumbromat und Bromwasserstoff.

Die Reaktion wurde in einer konzentrischen, zylindrischen 250 ml-4-Hals-Belichtungsapparatur (Glas) mit Magnetkreuz, Intensivkühler, Schlauchpumpe, PT100-Wärmefühler und Stickstoffüberlagerung durchgeführt. Im Zentrum des Zylinders befand sich eine UV-Tauchlampe (TQ150, Original Hanau) die mittels eines Kryostaten (Lauda RM6, Ethanol /Wasser-Gemisch 50:50) auf -6 bis -10°C gekühlt wurde.

In der Belichtungsapparatur wurden 10,30 g (67,9 mmol) 99,5% iges Natriumbromat in 12,5 ml Wasser suspendiert und unter Rühren teilweise gelöst. Die Suspension wurde mit Stickstoff überlagert. Es wurden 45,40 g (202,0 mmol) 4-Chloro-1-(4-isopropyl-phenyl)-butan-1-on (98,4 Fl.-%ig, GC) und 85 ml Dichlormethan zugegeben, wobei sich das Edukt sofort auflöste. Die gerührte Emulsion / Suspension wurde mit Argon durchperlt um gelösten Sauerstoff auszutreiben und dann mit einem Eis / Kochsalzbad (-15°C) auf-7°C gekühlt. Die UV-Lampe wurde angeschaltet und per Schlauchpumpe 25,5 ml (225,0 mmol) 48%ige wässrige Bromwasserstofflösung innerhalb 15 Minuten zudosiert, wobei die Reaktionstemperatur auf maximal +0,5°C anstieg. Direkt nach Zutropfende war der Natriumbromat-Feststoff vollständig verschwunden und die flüssigen Phasen waren nicht mehr braun gefärbt. Nach Zutropfende wurde noch 2 Minuten nachbelichtet, dann die Lampe abgestellt. Das flüssige Zweiphasengemisch wurde in einen Scheidetrichter überführt, die Phasen getrennt und die untere, organische Phase noch mit 3 x 25 ml Wasser gewaschen. Die organische Phase wurde am Rotationsverdampfer unter verminderten Druck unter azeotroper Trocknung eingeengt, der ölige Rückstand im Hochvakuum getrocknet. Beim Animpfen erfolgte rasche, vollständige Kristallisation. Erhalten wurden 61,05 g farblose Kristalle . ..

GC-Analyse: 2,1 Flächen% Edukt, 95,0 FI.% Produkt, 0,9 FI.% Dibromid.

61,05 g (95,0 FI.% = 58,0 g 100%iges Produkt) entsprechen einer Ausbeute von 94,6% d.Th.. Die Spektren entsprachen den in Beispiel 1A beschriebenen Daten.

Auswertung des ¹H-NMR-Integrals ergab 93,1 mol% gewünschtes Bromid,1,9 mol% Olefin, 2,7 mol% Dibromid und 2,3 mol% unumgesetztes Edukt.

### Beispiele 3 -10:

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Bromids (VIII, Y = Cl) in Supersäure oder in konzentrierter Schwefelsäure bei 40°C in Anwesenheit von präformiertem Kupfer(I)-tricarbonyl-lon als Additiv.

In jeden der acht Mini-Autoklaven (A1 bis A8) wurden 3,0 mg (0,0419 mmol, 22,8 mol% bezogen aufs Edukt) Cu₂O eingewogen. Die Miniautoklaven wurden in den Reaktorblock eingebaut und verschlossen. Die Autoklaven wurden sekuriert und im Argon-Gegenstrom, mittels inertisierter GC-Vials, mit 0,5 ml des jeweiligen Lösungsmittels befüllt (A1 bis A6 mit 98%iger Trifluormethansulfonsäure CF₃SO₃H der Firma Aldrich; A7 und A8 mit 96%iger Schwefelsäure der Firma Merck Darmstadt). Die Miniautoklaven wurden mit je einem gasdichten Septum verschlossen, erneut sekuriert und das Reaktionsgas Kohlenmonoxid bis zum gewünschten Druck aufgepresst (5 bar bei A1 und A2, 25 bar bei A3 und A 4, 40 bar bei A5 und A6, 60 bar bei A7 und A8). Der Reaktionsblock wurde unter magnetischem Rühren der Mini-Autoklaven (200 Umdrehungen/ Min) auf die Reaktionstemperatur von 40 °C aufgeheizt. In der folgenden 30 minütigen Präformierungsphase wurde durch Reaktion des Cu₂O mit CO in situ das Additiv [Cu(CO)₃]⁺ gebildet. In diesem Zeitraum wurde die Eduktlösung vorbereitet. Dazu wurden 1,12 g (3,68 mmol) 1-[4-(1-Brom-1-methyl-ethyl)-phenyl]-4-chlor-butan-1-on (aus Beispiel 2) in ein 2,5 ml Meßkölbchen eingewogen und mit etwa 1,4 ml Tetrachlorkohlenstoff bis zur Marke aufgefüllt, unter Bildung einer klaren Lösung. Nach Ablauf der Präformierungsphase wurden jeweils 125 µl (0,184 mmol) der Eduktlösung mittels einer druckfesten Glasspritze manuell innerhalb jeweils 1 Minute durch das Septum in jeden der acht Mini-Autoklaven eingespritzt. Nach erfolgter Eduktzugabe begann die Reaktionszeit von 4,0 Stunden. Nur zu Beginn der Reaktion wurde das CO-Gas genau bis zum Solldruck aufgedrückt. Im Verlauf der Carbonylierung fällt der Autoklaveninnendruck durch CO-Verbrauch etwas ab. Die Versuchsführung ist also nicht isobar. Nach Ablauf der Reaktionszeit wurde der Reaktorblock auf 25°C abgekühlt und entspannt. Die Autoklaven wurden ausgebaut und die Ansätze in je 2,0 ml Eiswasser aufgenommen. Die wässrige trübe Mischung wurde jeweils mit 2 x 2,0 ml Tetrachlorkohlenstoff extrahiert. Die organische Phase (unten) wurde jeweils quantitativ abgetrennt und mit Tetrachlorkohlenstoff auf genau 10 ml aufgefüllt. Von der so erhaltenen klaren Lösung wurde genau 1,0 ml in ein GC-Vial pipettiert. Die Probe wurde im Stickstoffstrom bei RT getrocknet und der erhaltene Rückstand in Acetonitril gelöst. Die Lösung wurde mittels HPLC analysiert (Säule: Zorbax Eclipse XDB-C8 150 x 4,6 mm; Temperatur:25 °C, Solvent A: 20 mM Triethylamin / Essigsäure-Puffer pH 7,0 ; Solvent B: 100% Acetonitril; lineares Gradientenprogramm: t = 0 A:B = 90:10, t = 15 min A:B =10:90; t = 25 min A:B = 10:90; Fluss: 1 ml / min; Detektion: UV bei 254 nm; Injektionsvolumen: 5,0 µl). Die Ausbeuten wurden aus den Peakflächen der erwarteten Carbonsäure (tᵣₑₜ = 7,26 min) nach der Methode des externen Standards berechnet, wobei die gemessenen Peakflächen mit einer Kalibrierungsgerade verglichen wurden. Die Kalibrierungsgerade wurde zuvor aus definierten Mengen der authentischen Carbonsäure ermittelt. Reaktionsparameter und berechnete Ausbeuten sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [% d.Th.] |
|---|---|---|---|---|---|---|
| A1 | Bromid 0,184 | Cu₂O | CF₃SO₃H | 5 | 40 | 47 |
| | | 0,0419 | | | | |
| A2 | Bromid 0,184 | Cu₂O | CF₃SO₃H | 5 | 40 | 40 |
| | | 0,0419 | | | | |
| A3 | Bromid 0,184 | Cu₂O | CF₃SO₃H | 25 | 40 | 72 |
| | | 0,0419 | | | | |
| A4 | Bromid 0,184 | Cu₂O | CF₃SO₃H | 25 | 40 | 73 |
| | | 0,0419 | | | | |
| A5 | Bromid 0,184 | Cu₂O | CF₃SO₃H | 40 | 40 | 71 |
| | | 0,0419 | | | | |
| A6 | Bromid 0,184 | Cu₂O | CF₃SO₃H | 40 | 40 | 61 |
| | | 0,0419 | | | | |
| A7 | Bromid 0,184 | Cu₂O | H₂SO₄ | 60 | 40 | 0 |
| | | 0,0419 | | | | |
| A8 | Bromid 0,184 | Cu₂O | H₂SO₄ | 60 | 40 | 0 |
| | | 0,0419 | | | | |

### Beispiele 11 -18:

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Bromids (VIII, Y = Cl) in Supersäure bzw. in konz. Schwefelsäure bei 40°C in An- bzw. Abwesenheit von präformiertem Kupfer(I)-tricarbonyl-lon als Additiv

Die Carbonylierungen wurden wie bei den Beispielen 3 -10 durchgeführt. In den Autoklaven A1-A4 wurde 96%iges Schwefelsäure, in den Autoklaven A5-A8 98%ige Trifluormethansulfonsäure eingesetzt. In den Autoklaven A1, A2, A5 und A6 wurde ohne Cu₂O-Zusatz gearbeitet. Bei allen Ansätzen wurde das Edukt, gelöst in CCl₄, bei Reaktionstemperatur und CO-Druck zudosiert. Die Ergebnisse sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [% d. Th.] |
|---|---|---|---|---|---|---|
| A1 | Bromid 0,184 | --- | H₂SO₄ | 40 | 40 | 0 |
| A2 | Bromid 0,184 | --- | H₂SO₄ | 40 | 40 | 0 |
| A3 | Bromid 0,184 | Cu₂O | H₂SO₄ | 40 | 40 | 0 |
| | | 0,0419 | | | | |
| A4 | Bromid 0,184 | Cu₂O | H₂SO₄ | 40 | 40 | 0 |
| | | 0,0419 | | | | |
| A5 | Bromid 0,184 | --- | CF₃SO₃H | 60 | 40 | 60 |
| A6 | Bromid 0,184 | --- | CF₃SO₃H | 60 | 40 | 62 |
| A7 | Bromid 0,184 | Cu₂O | CF₃SO₃H | 60 | 40 | 66 |
| | | 0,0419 | | | | |
| A8 | Bromid 0,184 | Cu₂O | CF₃SO₃H | 60 | 40 | 56 |
| | | 0,0419 | | | | |

### Beispiele 19 - 26:

### Synthese von 2-[4-(4-Chloro-butyryl)-phenyl]-2-methyl-propionsäure (Formel 1, R¹ = R² = Methyl; R³ = 4-Chlorbutyryl) per Carbonylierung des Bromids (VIII, Y = Cl) in Supersäure bei 0°C in An- bzw. Abwesenheit von präformiertem Kupfer(1)-tricarbonyl-lon als Additiv

Die Carbonylierungen wurden wie bei den Beispielen 3 -10, jedoch unter Kühlung des Reaktorblocks auf 0 °C durchgeführt. In den Autoklaven A1, A2, A5 und A6 wurde ohne Cu₂O-Zusatz gearbeitet. Bei allen Ansätzen wurde das Edukt, gelöst in CCl₄, bei 0°C und CO-Druck zudosiert. Die Ergebnisse sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [% d. Th.] |
|---|---|---|---|---|---|---|
| A1 | Bromid 0,184 | --- | CF₃SO₃H | 25 | 0 | 69 |
| A2 | Bromid 0,184 | --- | CF₃SO₃H | 25 | 0 | 60 |
| A3 | Bromid 0,184 | Cu₂O | CF₃SO₃H | 25 | 0 | 70 |
| | | 0,0419 | | | | |
| A4 | Bromid 0,184 | Cu₂O | CF₃SO₃H | 25 | 0 | 52 |
| | | 0,0419 | | | | |
| A5 | Bromid 0,184 | --- | CF₃SO₃H | 40 | 0 | 75 |
| A6 | Bromid 0,184 | --- | CF₃SO₃H | 40 | 0 | 74 |
| A7 | Bromid 0,184 | Cu₂O | CF₃SO₃H | 40 | 0 | 60 |
| | | 0,0419 | | | | |
| A8 | Bromid 0,184 | Cu₂O | CF₃SO₃H | 40 | 0 | 50 |
| | | 0,0419 | | | | |

Bei allen Ansätzen hatte der HPLC-Peak der gewünschten Carbonsäure >95Fl.% aller Peaks. Beim Produkt in den Autoklaven A5 und A6 hatte der HPLC-Peak der gewünschten Carbonsäure >98 Fl.-% aller Peaks.

Die gepoolten CCl₄-Extrakte der in Wasser aufgenommenen Carbonylierungsreaktionen aus Autoklaven A1 bis A8 wurden mit Wasser gewaschen, das organische Lösungsmittel unter verminderten Druck entfernt und der Rückstand im HV getrocknet. Erhalten wurden 250 mg (0,93 mmol, 64% d. Th.) kristalliner Feststoff, dessen ¹H-NMR-Spektrum, HPLC-Retention und UV-Spektrum identisch mit denen der authentischen Referenzsubstanz waren. ¹H-NMR (CDCl₃): δ = 1,63 (s, 6H, 2 x CH₃), 2,23 (qui, 2H, CH₂), 3,16 (t, 2H, CH₂), 3,67 (t, 2H, CH₂), 7,50 (∼d, 2H, arom.-H), 7,96 (∼d, 2H, ar m.-H). UV (Dioden-Array Spektrum): λₘₐₓ = 261 und 207 nm.

### Beispiel 27:

### Synthese von 1-[4-(1-Hydroxy-1-methyl-ethyl)-phenyl]-4-chlor-butan-1-on (Formel X, Y = Cl) per benzylischer Oxidation von (III) (R¹ = R² = Methyl, R³ = 4-Chlorbutyryl) mit Sauerstoff

In einem 100 ml Dreihals-Rundkolben mit mechanischem Rührer [Schliffe und Rührwelle mit Poly(monochlortrifluorethylen) abgedichtet], Rückflusskühler mit einem aufgesetztes Sauerstoff-gefülltem Ballon (Naturlatex), sowie einem Innenthermometer wurden 4,500 g (19,6 mmol) 4-Chlor-1-(4-isopropyl-phenyl)-butan-1-on (97,9%ig, aus Referenzbeispiel 2), 659 mg (3,92 mmol, 0,2 Äquivalente) N-Hydroxyphthalimid (NHP, 97%ig, Aldrich) und 99 mg (0,392 mmol, 0,02 Äquiv.) Kobalt(II)acetat Tetrahydrat (99%ig, Merck) in 25 ml Acetonitril (Roth) gelöst. Das Reaktionsgemisch färbte sich beim Rühren unter der geschlossenen SauerstoffAtmosphäre rasch braun. Es wurde in einem Ölbad auf 40°C erwärmt. Nach 7 Stunden färbte sich die Lösung grün.

HPLC (System wie in Beispielen 3 -10) einer Probe zeigte zu diesem Zeitpunkt neben NHP (tᵣₑₜ 3,9 min) 44 Fl.-% des erwarteten Alkohols (tᵣₑₜ 11,0 min), 43 Fl.-% des entsprechenden Hydroperoxids (tᵣₑₜ 11,8 min), 4 Fl.-% des Edukts (tᵣₑₜ = 15,1 min) und 7 Fl.-% eines Nebenprodukts (tᵣₑₜ 17,2 min) an. Nach insgesamt 9stündigem Rühren bei 40°C wurde Heizung und Rührung abgestellt und das Reaktionsgemisch über Nacht bei RT stehen gelassen. HPLC einer Probe zeigte jetzt neben NHP 59 Fl.-% des Alkohols, 32 Fl.-% des Hydroperoxids, 0,5 Fl.-% Edukt und 7 Fl.-% des Nebenprodukts an. Die Lösung wurde auf die 3-fache Menge Wasser gegossen und mit 3 x 30 ml Dichlormethan extrahiert. Die vereinigten organischen Extrakte wurden mit 2 x 15 ml Wasser gewaschen, unter verminderten Druck eingeengt und der ölige Rückstand im HV getrocknet. Er wurde durch 434 g Kieselgel 60 (Merck, 0,04 - 0,063 mm) Mitteldruck-chromatographiert (Fluss 80 ml/min), wobei zunächst mit 1,8 L n-Heptan /Ethylacetat 90:10 entwickelt und dann mit 1 L 85:15, gefolgt von 1 L 80:20, zunächst 100 mg (0,4 mmol) des Hydroperoxids, gefolgt, nach einigen Mischfraktionen, von 2,98 g (12,38 mmol, 63% d. Th.) des gewünschten Alkohols eluiert wurden. Spektren und physikalische Eigenschaften des Alkohols waren identisch mit denen des Produkts aus Beispiel 28.

Das Hydroperoxid hat folgende Spektraldaten:
¹H-NMR (CDCl₃): δ = 1,62 (s, 2 x CH₃, 6H), 2,22 (t, 2H, CH₂), 3,17 (t, 2H, CH₂), 3,67 (t, 2H, CH₂), 7,57 (~d, 2H, arom.-H), 7,75 (br s, 1H, OOH), 7,97 (∼d, 2H, arom.-H). ¹³C-NMR (CDCl₃): δ = 26,10 (2 x CH₃), 26,78 (CH₂), 35,33 (CH₂-CO), 44,65 (CH₂-Cl), 83,79 (C-OOH), 125,74 (2 x arom.-CH), 128,32 (2 x arom.-CH), 135,76 (arom.-C), 140,27 (arom.-C), 150,55 (C=0). IR (Flüssigkeitsfilm): ν = 3600 - 3200 (br, OO-H), 1673 (C=O), 1266,1227,907,730 cm⁻¹. MS (ESI+): m/z = 259,12 (M+H⁺ mit ³⁷Cl), 257,10 (M+H⁺ mit ³⁵Cl).

### Beispiel 28:

### Synthese von 1-[4-(1-Hydroxy-1-methyl-ethyl)-phenyl]-4-chlor-butan-1-on (Formel X; Y = Cl) durch Hydrolyse des Bromids (Formel VIII, Y = Cl) in pH7-Puffer

Eine pH7-Pufferlösung wurde hergestellt, indem 2,02 g (20 mmol) Triethylamin in 1 L Millipor-Wasser gelöst und dann Eisessig bis pH 7,00 zugegeben wurde.

In einem 2 L Rundkolben wurden unter Stickstoff 19,1 g (61,4 mmol) 1-[4-(1-Brom-1-methyl-ethyl)-phenyl]-4-chlor-butan-1-on (Formel VIII, Y = Cl) (97,5%ig, Beispiel 1) in 765 ml Acetonitril gelöst, dann 383 ml der Pufferlösung zugegeben. Man ließ die Lösung im verschlossenen Kolben 1 Tag bei RT und 2 Tage im Kühlschrank bei +3°C stehen. Sie wurde dann unter verminderten Druck auf ein Drittel des ursprünglichen Volumens eingeengt und mit 1 x 100 und 2 x 50 ml Dichlormethan extrahiert. Die vereinigten Extrakte wurden mit 50 ml Wasser gewaschen und die wässrige Phase mit 50 ml Dichlormethan zurückextrahiert. Die vereinigten Dichlormethan-Phasen wurden unter verminderten Druck eingeengt und das zurückbleibende Öl im HV getrocknet. Es wurde, gelöst in 30 ml n-Heptan / Ethylacetat 8:2 plus 2 ml Dichlormethan auf eine Mitteldruck-Chromatographiesäule (Durchmesser 9,5 cm, Länge 48 cm) aufgegeben, die 1,9 kg Kieselgel 60 (Merck, 0,04 - 0,063 mm) enthielt, welches zuvor mit 6 L Heptan / Ethylacetat 9:1 konditioniert worden war. Entwicklung und Elution erfolgten bei einem Fluss von 160 ml / min mit einem Heptan / Ethylacetat- Gradienten (2L 90:10, 6L 80:20, 5 L 75:25, 3 L 70:30,1 L 60:40, 3 L 55:45, 5 L 50:50). Es wurden Fraktionen von 150 ml gesammelt. Das reine Produkt eluierte in Fraktionen 54 - 75. Die Lösungsmittel wurden unter verminderten Druck entfernt und der Rückstand im HV unter Rühren getrocknet. Ausbeute: 11,1 g (46,1 mmol, 75% d. Th.) eines Öls.

Gemäß HPLC (System wie in Beispielen 3-10) hatte der Alkohol eine Reinheit von 99,2 Fl.-% (tᵣₑₜ 10,9 min). GC (System wie in Referenzbeispiel 2): tᵣₑₜ 12.0 Min Der Alkohol ist auch in reinem Zustand ein leicht trübes farbloses Öl, das im Tiefkühlschrank kristallisiert, aber beim Erwärmen wieder in der Nähe von Raumtemperatur schmilzt. Es wird unter Argon im Tiefkühlschrank gelagert. Bei Lagerung bei Raumtemperatur verfärbt es sich nach einigen Tagen gelblich. ¹H-NMR (CDCl₃): δ = 1,60 (s, 6H, 2 x CH₃), 2,03 (br s, 1H, OH), 2,22 (qui, 2H, CH₂), 3,17 (t, 2H, CH₂), 3,67 (t, 2H, CH₂), 7,59 (∼d, 2H, arom.-H), 7,94 (∼d, 2H, arom.-H). ¹³C-NMR (CDCl₃): d = 26,81 (CH₂), 31,68 (2 x CH₃), 35,29 (CH₂-CO), 44,67 (CH₂-Cl), 72,54 (C-OH), 124,76 (2 x arom.-CH), 128,12 (2 x arom.-CH), 135,21 (arom.-C), 154,62 (arom.-C-CO), 198,69 (C=O).

### Beispiele 29 - 36:

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Alkohols (Formel X, Y = Cl) in Supersäure bei 30°C in Anwesenheit von präformiertem Kupfer(I)-trcarbonyl-lon als Additiv

In jeden der acht Mini-Autoklaven (A1 bis A8) wurden 3,0 mg (0,0419 mmol, 20,1 mol% bezogen aufs Edukt) Cu₂O eingewogen. Die Miniautoklaven wurden in den Reaktorblock eingebaut und verschlossen. Die Autoklaven wurden sekuriert und im Argon-Gegenstrom, mittels inertisierter GC-Vials, mit jeweils 0,5 ml 98%iger Trifluormethansulfonsäure CF₃SO₃H (Aldrich) befüllt. Die Mini-Autoklaven wurden mit je einem gasdichten Septum verschlossen, erneut sekuriert und das Reaktionsgas Kohlenmonoxid bis zum gewünschten Druck aufgepresst (5 bar bei A1 und A2, 25 bar bei A3 und A 4, 40 bar bei A5 und A6, 60 bar bei A7 und A8). Der Reaktionsblock wurde unter magnetischem Rühren der Mini-Autoklaven (200 Umdrehungen/ Min) auf die Reaktionstemperatur von 30°C aufgeheizt. In der folgenden 30minütigen Präformierungsphase wurde durch Reaktion des Cu₂O mit CO in situ das Additiv [Cu(CO)₃]⁺ gebildet. In diesem Zeitraum wurde die Eduktlösung vorbereitet. Dazu wurden 1,00 g (4,15 mmol) 1-[4-(1-Hydroxy-1-methyl-ethyl)-phenyl]-4-chlor-butan-1-on (aus Beispiel 28) in ein 2,5 ml Meßkölbchen eingewogen und mit etwa 1,5 ml Tetrachlorkohlenstoff bis zur Marke aufgefüllt, unter Bildung einer klaren Lösung. Nach Ablauf der Präformierungsphase wurden jeweils 125 µl (0,208 mmol) der Eduktlösung mittels einer druckfesten Glasspritze manuell innerhalb jeweils 1 Minute durch das Septum in jeden der acht Mini-Autoklaven eingespritzt. Nach erfolgter Eduktzugabe begann die Reaktionszeit von 4,0 Stunden. Nur zu Beginn der Reaktion wurde das CO-Gas genau bis zum Solldruck aufgedrückt. Im Verlauf der Carbonylierung fällt der Autoklaveninnendruck durch CO-Verbrauch etwas ab. Die Versuchsführung ist also nicht isobar. Nach Ablauf der Reaktionszeit wurde der Reaktorblock auf 25°C abgekühlt und entspannt. Aufarbeitung und Analyse erfolgten wie für Beispiele 3 -10 beschrieben. Reaktionsparameter und berechnete Ausbeuten sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [% d. Th.] |
|---|---|---|---|---|---|---|
| A1 | Alkohol 0,208 | Cu₂O | CF₃SO₃H | 5 | 30 | 53 |
| | | 0,0419 | | | | |
| A2 | Alkohol 0,208 | Cu₂O | CF₃SO₃H | 5 | 30 | 54 |
| | | 0,0419 | | | | |
| A3 | Alkohol 0,208 | Cu₂O | | 25 | 30 | 58 |
| | | 0,0419 | | | | |
| A4 | Alkohol 0,208 | Cu₂O | CF₃SO₃H | 25 | 30 | 56 |
| | | 0,0419 | | | | |
| A5 | Alkohol 0,208 | Cu₂O | | 40 | 30 | 59 |
| | | 0,0419 | | | | |
| A6 | Alkohol 0,208 | Cu₂O | CF₃SO₃H | 40 | 30 | 54 |
| | | 0,0419 | | | | |
| A7 | Alkohol 0,208 | Cu₂O | | 60 | 30 | 58 |
| | | 0,0419 | | | | |
| A8 | Alkohol 0,208 | Cu₂O | CF₃SO₃H | 60 | 30 | 52 |
| | | 0,0419 | | | | |

### Beispiele 37 - 44:

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Alkohols (Formel X; Y = Cl) in Supersäure bei 40°C in Abwesenheit von Additiv

Die Carbonylierungen wurden wie bei den Beispielen 29 - 36, jedoch bei 40°C und in Abwesenheit von Additiv durchgeführt. Die Ergebnisse sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [%d.Th.] |
|---|---|---|---|---|---|---|
| A1 | Alkohol 0,208 | --- | CF₃SO₃H | 5 | 40 | 43 |
| A2 | Alkohol 0,208 | --- | CF₃SO₃H | 5 | 40 | 47 |
| A3 | Alkohol 0,208 | --- | CF₃SO₃H | 25 | 40 | 51 |
| A4 | Alkohol 0,208 | --- | CF₃SO₃H | 25 | 40 | 52 |
| A5 | Alkohol 0,208 | --- | CF₃SO₃H | 40 | 40 | 53 |
| A6 | Alkohol 0,208 | --- | CF₃SO₃H | 40 | 40 | 53 |
| A7 | Alkohol 0,208 | --- | CF₃SO₃H | 60 | 40 | 51 |
| A8 | Alkohol 0,208 | --- | CF₃SO₃H | 60 | 40 | 50 |

### Beispiele 45 - 52:

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Alkohols (Formel X, Y = Cl) in Supersäure bzw. in konz. Schwefelsäure bei 40°C unter 87 bar CO-Druck

In die Mini-Autoklaven A3, A4, A7 und A8) wurden jeweils 3,0 mg (0,0419 mmol, 20,1 mol bezogen aufs Edukt) Cu₂O eingewogen, die Mini-Autoklaven A1, A2, A5 und A6 wurden ohne Additiv belassen. Die acht Miniautoklaven wurden in den Reaktorblock eingebaut und verschlossen. Die Autoklaven wurden sekuriert, auf 0°C abgekühlt und im Argon-Gegenstrom, mittels inertisierter GC-Vials, mit 0,5 ml des jeweiligen Lösungsmittels (96%ige Schwefelsäure bei A1-A4, 98%ige Trifluormethansulfonsäure CF₃SO₃H bei A5-A8) und mit 125 µl (0,208 mmol) der Eduktlösung (in CCl₄) befüllt. Die Präformierung der Cu2O enthaltenden Ansätze entfiel bei diesen Ansätzen wegen des starren, automatisierten Reaktionsablaufs. Die Mini-Autoklaven wurden verschlossen, erneut sekuriert und 87 bar des Reaktionsgases Kohlenmonoxid aufgepresst. Der Reaktionsblock wurde unter magnetischem Rühren der Mini-Autoklaven (200 Umdrehungen/ Min) auf 40°C aufgeheizt. Die Aufheizzeit betrug 15 Minuten. Dann wurde 4 Stunden bei 40°C gerührt. Im Verlauf der Carbonylierung fällt der Autoklaveninnendruck durch CO-Verbrauch etwas ab. Die Versuchsführung ist also nicht isobar. Nach Ablauf der Reaktionszeit wurde der Reaktorblock auf 25°C abgekühlt und entspannt. Aufarbeitung und Analyse erfolgten wie für Beispiele 3 -10 beschrieben. Reaktionsparameter und berechnete Ausbeuten sind in der Tabelle zusammengefasst:

| Autoklaven-Nr. | Edukt [mmol] | Additiv [mmol] | Säure 0,5 ml | CO-Druck [bar] | Temp. [°C] | Ausbeute (I) [% d. Th.] |
|---|---|---|---|---|---|---|
| A1 | Alkohol 0,208 | --- | H₂SO₄ | 87 | 40 | 0 |
| A2 | Alkohol 0,208 | --- | H₂SO₄ | 87 | 40 | 0 |
| A3 | Alkohol 0,208 | Cu₂O | H₂SO₄ | 87 | 40 | 0 |
| | | 0,0419 | | | | |
| A4 | Alkohol 0,208 | Cu₂O | H₂SO₄ | 87 | 40 | 0 |
| | | 0,0419 | | | | |
| A5 | Alkohol 0,208 | --- | CF₃SO₃H | 87 | 40 | 53 |
| A6 | Alkohol 0,208 | --- | CF₃SO₃H | 87 | 40 | 52 |
| A7 | Alkohol 0,208 | Cu₂O | CF₃SO₃H | 87 | 40 | 53 |
| | | 0,0419 | | | | |
| A8 | Alkohol 0,208 | Cu₂O | CF₃SO₃H | 87 | 40 | 51 |
| | | 0,0419 | | | | |

### Beispiel 53:

### Isolierung des Produkts 2-[4-(4-Chloro-butyryl)-phenyl]-2-methyl-propionsäure (Formel I, R¹= R² = Methyl; R³ = 4-Chtorbutyryl) aus den Carbonylierungen Beispiele 29 - 52

Die gepoolten CCl₄-Extrakte der in Wasser aufgenommenen Carbonylierungsreaktionen aus Beispielen 29 -44 und 49 - 52 wurden mit Wasser gewaschen, das organische Lösungsmittel unter verminderten Druck entfernt und der Rückstand im HV getrocknet. Erhalten wurden 622 mg (2,31 mmol, 56% d. Th.) kristalliner Feststoff, dessen ¹H-NMR-Spektrum, HPLC-Retention und UV-Spektrum identisch mit denen der authentischen Referenzsubstanz waren.

### Beispiel 54:

### Synthese des Alkens 1-[4-(2-Propenyl)-phenyl]-4-chlor-butan-1-on [Formel II, R¹ = Methyl, R³ = 4-Chlorbutyryl, R⁴ = Methylen] durch Dehydrobromierung des benzylischen Bromids

20,6 g (67,8 mmol) 1-[4-[1-Brom-1-methyl-ethyl)-phenyl]-4-chlor-butan-1-on [Formel VIII, Y = Cl) (97,5%ig, Beispiel 1) wurden einem Gemisch von 14,8 g (170 mmol) Lithiumbromid und 7,6 g (102 mmol) Lithiumcarbonat in 60 ml DMF zugesetzt und 1 Tag bei RT gerührt. Der Suspension wurde Wasser zugesetzt, bis eine klare Lösung entstanden war. Diese wurde mit 4 x 50 ml n-Heptan extrahiert und die vereinigten Heptan-Extrakte mit 3 x 50 ml Wasser gewaschen. Die Heptan-Phase wurde unter verminderten Druck eingeengt und der Rückstand im HV getrocknet (16,6 g Rohprodukt). Mitteldruck-Chromatographie durch 900 g Kieselgel 60 (Merck, 0,04 - 0,063 mm) mit einem n-Heptan / Diethylether - Gradienten (99:1 bis 90:10) ergab 10,7 g (48,0 mmol, 71% d. Th.) farblose, schuppige Kristalle, Schmp. 54-55°C. ¹H-NMR (CDCl₃): δ = 2,19 (s, 3H, CH₃), 2,22 (qui, 2H, CH₂), 3,18 (t, 2H, CH₂), 3.69 (t, 2H, CH₂), 5,21 (s, 1H, =CH), 5,48 (s, 1H, =CH), 7,55 (∼d, 2H, arom.-H), 7,94 (∼d, 2H, arom.-H).

### Beispiele 55 - 70

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Bromids (VIII, Y = Cl) in Trifluormethansulfonsäure in Gegenwart zugesetzter, definierter Wassermengen

Die verwendeten Miniautoklaven, Gefäße, HPLC-Gläschen und Spritzen wurden im Trockenschrank bei 60°C unter verminderten Druck getrocknet. 8 HPLC-Gläschen wurden in einer Glovebox mit jeweils 75 mg des Bromids (VIII, Y = Cl; 98.5% ig) gelöst in je 150 µl CCl₄ befüllt. In acht Glasgefäßen wurde Trifluormethansulfonsäure der Firma Central Glass (Reinheit >99,5 Gew.-%, Wassergehalt 160 ppm, entsprechend einem Wassergehalt von 4 mol% bezogen aufs Edukt VIII (Y = Cl) exakt soviel Wasser zugesetzt, dass sie anschließend Wassergehalte von 4 mol%, 23 mol%, 45 mol%, 90 mol%, 100 mol%, 200 mol%, 500 mol% bzw. 2000 mol% bezogen aufs Edukt VIII (Y = Cl) hatten. Die zugehörigen Gew.-% Wasser können der folgenden Tabelle entnommen werden. In einer Glovebox wurden unter Argon mittels einer Spritze jeweils 500 µl dieser wässrigen Säure in acht HPLC-Vials abgefüllt.

Das Rack mit den acht 2 ml-Miniautoklaven (A1 bis A8) wurde mit Argon inertisiert und die Säure aus den acht HPLC-Vials mit Argon und gegen Argonstrom in die acht Miniautoklaven übergedrückt. Bei -5°C wurde sekuriert, dann CO aufgedrückt und die Reaktionstemperatur von 0°C eingestellt. Der Druck wurde auf 40 bar eingestellt. Die CCl₄-Lösungen des Bromids wurden mittels einer gasdichten Spritze eingespritzt (Zugabezeit etwa 30 Sekunden für jede Lösung) und dann 20 Stunden reagieren gelassen. 8 Reagenzgläser wurden mit je 5 ml CCl₄ befüllt. Die Miniautoklaven wurden entspannt und geöffnet. Der Inhalt jedes der Miniautoklaven wurden mittels eines Trichters in je eines der Reagenzgläser gegossen. Es wurde jeweils mit 2 ml Eiswasser übergespült und dann jeweils mit 10 ml CCl₄ Autoklav und Trichter nachgespült. Die Phasen wurden durchgeschüttelt und sich trennen gelassen. Dann wurde jeweils 0,5 ml der organischen Phase entnommen und im Stickstoffstrom eingedunstet. Der Rückstand wurde jeweils in 2,5 ml Acetonitril aufgenommen und im HPLC bei einer Verdünnung von 1:30 analysiert. Die Ausbeute wurde aus der Peakfläche der Carbonsäure unter Verwendung einer Eichkurve berechnet.

| Autoklaven-Nr. | Edukt [mmol] | Säure 0,5 ml New.-% H₂O | gesamter Wassergehalt [bezogen auf Edukt] | CO-Druck [bar] | Temp. [°C] | Ausbeute (I) [% d. Th.] |
|---|---|---|---|---|---|---|
| A1 | Bromid 0,243 | CF₃SO₃H | 4 mol% | 40 | 0 | 16 |
| | | 0,0296 H₂O | | | | |
| A2 | Bromid 0,243 | CF₃SO₃H | 23 mol% | 40 | 0 | 50 |
| | | 0,118% H₂O | | | | |
| A3 | Bromid 0,243 | CF₃SO₃H | 45 mol% | 40 | 0 | 74 |
| | | 0,235% H₂O | | | | |
| A4 | Bromid 0,243 | CF₃S0₃H | 90 mol% | 40 | 0 | 93 |
| | | 0,471% H₂O | | | | |
| A5 | Bromid 0,243 | CF₃SO₃H | 100 mol% | 40 | 0 | 95 |
| | | 0,522% H₂O | | | | |
| A6 | Bromid 0,243 | CF₃SO₃H | 200 mol% | 40 | 0 | 99 |
| | | 1,044% H₂O | | | | |
| A7 | Bromid 0,243 | CF₃SO₃H | 500 mol% | 40 | 0 | 87 |
| | | 2,610% H₂O | | | | |
| A8 | Bromid 0,243 | CF₃SO₃H | 2000 mol% | 40 | 0 | 0 |
| | | 10,44% H₂O | | | | |

Mit einem weiteren Rack von 8 Miniautoklaven wurde ein identisches Experiment durchgeführt, mit dem einzigen Unterschied, dass die Carbonylierungen bereits nach 5 Stunden, anstatt nach 20 Stunden, abgebrochen wurden. Die Ergebnisse sind in der folgenden Tabelle zusammengefasst. Wegen eines Defekts konnten die Ausbeuten in den Autoklaven A5, A7 und A8 hier nicht ermittelt werden.

| Autoklaven-Nr. | Edukt [mmol] | Säure 0,5 ml Gew.-% H₂O | gesamter Wassergehalt [bezogen auf Edukt] | CO-Druck [bar] | Temp. [°C] | Ausbeute (I) [% d. Th.] |
|---|---|---|---|---|---|---|
| A1 | Bromid 0,243 | CF₃SO₃H | 4 mol% | 40 | 0 | 19 |
| | | 0,02% H₂O | | | | |
| A2 | Bromid 0,243 | CF₃SO₃H | 23 mol% | 40 | 0 | 45 |
| | | 0,118% H₂O | | | | |
| A3 | Bromid 0,243 | CF₃SO₃H | 45 mol% | 40 | 0 | 67 |
| | | 0,235% H₂O | | | | |
| A4 | Bromid 0,243 | CF₃SO₃H | 90 mol% | 40 | 0 | 91 |
| | | 0,471% H₂O | | | | |
| A5 | Bromid 0,243 | CF₃SO₃H | 100 mol% | 40 | 0 | nicht |
| | | 0,522% H₂O | | | | bestimmt |
| A6 | Bromid 0,243 | CF₃SO₃H | 200 mol% | 40 | 0 | 94 |
| | | 1,044% H₂O | | | | |
| A7 | Bromid 0,243 | CF₃SO₃H | 500 mol% | 40 | 0 | nicht |
| | | 2,610% H₂O | | | | bestimmt |
| A8 | Bromid 0,243 | CF₃SO₃H | 2000 mol% | 40 | 0 | nicht |
| | | 10,44% H₂O | | | | bestimmt |

Bei der Carbonylierung des Bromids der Formel (VIII) (Y = Cl) in Trifluormethansulfonsäure hängt die Vollständigkeit des Umsatzes des Edukts (VIII), sowie die erzielte Ausbeute und Reinheit der gewünschten Carbonsäure (I) in hohem Maße vom Wassergehalt der Trifluormethansulfonsäure ab.

Wenn trockene Trifluormethansulfonsäure [Wassergehalt 160 ppm, entsprechend einem Wassergehalt von 4 mol% bezogen aufs Edukt (VIII)] ohne weiteren Wasserzusatz zur Carbonylierung (0°C, 40 bar CO) eingesetzt wurde, so ergab es einen Umsatz von weniger als 50 % des Edukts (II). Die Carbonsäure (I) wurde mit einer Ausbeute von 16 % bis 19 % gebildet und die HPLC-Analyse der ungereinigten Carbonylierungslösung zeigte einen unsauberen Reaktionsverlauf an. Unter ansonsten identischen Reaktionsparametern konnte mit zunehmendem Wassergehalt der Trifluormethansulfonsäure der Umsatz und die Ausbeute der Carbonylierungsreaktion kontinuierlich gesteigert werden und man erhielt einen sauberen Reaktionsverlauf. Die folgende Tabelle fasst die Ausbeute in Abhängigkeit von der Wassermenge nochmals zusammen:

| | | | | | | | | | |
|---|---|---|---|---|---|---|---|---|---|
| Mol % Wasser, bezogen auf eingesetztes Edukt (VIII), in Trifluormethansulfonsäure | | 4 | 23 | 45 | 90 | 100 | 200 | 500 | 2000 |
| Ausbeute (% d.Th.) an Carbonsäure (I), | 20 Std. | 16 | 50 | 74 | 93 | 95 | 99 | 87 | 0 |
| bezogen auf eingesetztes Edukt (II) | 5 Std. | 19 | 45 | 67 | 91 | | 94 | | |

| | | | | | | | | | |
|---|---|---|---|---|---|---|---|---|---|
| % d. Th. bedeutet % der Theorie Std. bedeutet Stunden | | | | | | | | | |

In Anwesenheit von 100 - 200 mol% Wasser erzielte man bis zu 99% Umsatz des Edukts (VIII), sehr sauberen Reaktionsverlauf und bis zu 99% Ausbeute der Carbonsäure (I). Während die Ausbeute bei Anwesenheit von 500 mol% Wasser nur auf 87% abfiel, bewirkten nochmals deutlich höhere Wassergehalte ein völliges Zusammenbrechen der Carbonylierungsreaktion. Bei einem Wassergehalt von 2000 mol%, welcher in etwa dem Einsatz von Trifluormethansulfonsäure-Monohydrat entspricht, wurde keine Carbonsäure (I) mehr gebildet.

### Beispiel 71

Synthese von 2-[4-(4-Chloro-butyryl)-phenyl]-2-methyl-propionsäure (Formel I, R¹ R² = Methyl; R³ = 4-Chlorbutyryl) per präparativer Carbonylierung des Bromids (VIII, Y = Cl) in Trifluormethansulfonsäure in Gegenwart von 200 mol% Wasser bezogen auf Edukt.

Dosierung des Edukts in konzentrierter CCl4-Lösung; Aufarbeitung der Carbonylierungsreaktion mit wässriger Natriumcarbonat-lösung.

In einen 500 ml Hasteloy Büchi-Autoklaven mit Begasungsrührer wurden 100 ml Trifluormethansulfonsäure (Firma Central Glass Co., 99,5%ig, 0,02% Wasser) eingefüllt, der zuvor 1,72 ml Wasser zugesetzt worden war. Der Wassergehalt der Säure betrug somit 200 mol% bezogen auf das Edukt [Bromid der Formel (VIII)]. Der Autoklav wurde gasdicht verschlossen, Stickstoff aufgedrückt und entspannt, dann dreimal jeweils CO aufgedrückt, kräftig gerührt und wieder entspannt. Dann wurden 40 bar CO aufgedrückt, der Rührer auf eine Drehzahl von 1000 Umdrehungen / Min. und die Reaktionstemperatur auf 0 °C eingestellt. Mittels einer HPLC-Pumpe wurde eine Lösung von 15,7 g 94,9%igem 1-[4-(1-Brom-1-methyl-ethyl)-phenyl]-4-chlor-butan-1-on (49,07 mmol) in 19,0 ml Tetrachlorkohlenstoff innerhalb 3 Minuten bei 0 °C zudosiert und dann weitere 22 Stunden bei 0 °C unter 40 bar CO gerührt. Der Autoklav wurde entspannt und der Inhalt (150 ml gelbe, klare Lösung, auf deren Oberfläche sich eine CCl₄-Phase befand) abgelassen. Der Autoklav wurde mit 150 ml CCl₄ unter Rühren gespült und diese Spüllösung verworfen.

Die Trifluormethansulfonsäurelösung wurde innerhalb 15 Minuten unter Rühren auf 400 g Eis getropft, wobei sich die Reaktionslösung entfärbte, die Temperatur auf unter 0 °C absank und ein farbloser Niederschlag ausfiel. 50 ml Dichlormethan wurden unter Rühren zugegeben. Die untere, organische, blassgelbe Phase wurde von der oberen, wässrigen, farblosen Phase (pH 0,1) getrennt. Die wässrige Phase wurde mit weiteren 50 ml Dichlormethan extrahiert. Die vereinigten organischen Phasen (177,37 g) wurden mit 2 mal je 50 ml Eiswasser je 1 Minute gewaschen (letzte Waschlösung hatte pH 3,7). Die organische Phase wurde bei 0 °C mit 50 ml und dann ein weiteres Mal mit 25 ml eiskalter 1M wässriger Natriumcarbonatlösung extrahiert. Der letzte wässrige Extrakt hatte pH 10,5. Die vereinigten wässrigen Extrakte (pH 9,4) wurden mittels durchperlendem Stickstoff von restlichem Dichlormethan befreit und dann bei 0 bis +5 °C (Eiskühlung) mit 18 ml 30%iger Salzsäure unter Rühren angesäuert, wobei die Carbonsäure kristallin ausfiel. Der pH-Wert verharrte dabei längere Zeit bei pH 6,7 bis 6,5. Es wurde 0,5 Stunden im Eisbad nachgerührt. Der Niederschlag wurde abgesaugt, mit 50 ml Eiswasser gewaschen und bei Raumtemperatur über Nacht im Hochvakuum über Phosphorpentoxid im Exsiccator getrocknet. Man erhielt 12,7 g farblose Kristalle (47,26 mmol, 96,3% d.Th.). HPLC-Analyse der Carbonsäure ergab eine Reinheit von 99,4 FI.-%.

### Beispiel 72

Synthese von 2-[4-(4-Chloro-butyryl)-phenyl]-2-methyl-propionsäure (Formel I, R¹ R² = Methyl; R³ = 4-Chlorbutyryl) per präparativer Carbonylierung des Bromids (VIII, Y = Cl) in Trifluormethansulfonsäure in Gegenwart von 200 mol% Wasser bezogen auf Edukt.

Dosierung des Edukts in CH₂Cl₂-Lösung; Aufarbeitung der Carbonylierungsreaktion mit wässriger Natriumcarbonat-Lösung.

In einen 500 ml Hasteloy Büchi-Autoklaven mit Begasungsrührer wurden 100 ml Trifluormethansulfonsäure (Firma Central Glass Co., 99,5%ig, 0,02% Wasser) eingefüllt, der zuvor 1,72 ml Wasser zugesetzt worden war. Der Wassergehalt der Säure betrug somit 200 mol% bezogen auf das Edukt [Bromid der Formel (VIII)]. Der Autoklav wurde gasdicht verschlossen, Stickstoff aufgedrückt und entspannt, dann dreimal jeweils CO aufgedrückt, kräftig gerührt und wieder entspannt. Dann wurden 40 bar CO aufgedrückt, der Rührer auf eine Drehzahl von 1000 Umdrehungen / Min. und die Reaktionstemperatur auf 0 °C eingestellt. Mittels einer HPLC-Pumpe wurde eine Lösung von 15,08 g 94,9%igem 1-[4-(1-Brom-1-methyl-ethyl)-phenyl]-4-chlor-butan-1-on (47,13 mmol) in 19,0 ml Dichlormethan innerhalb 3 Minuten bei 0 °C zudosiert und dann weitere 22 Stunden bei 0 °C unter 40 bar CO gerührt. Der Autoklav wurde entspannt und der Inhalt (150 ml gelbe, klare Lösung) abgelassen. Der Autoklav wurde mit 170 ml Dichlormethan unter Rühren gespült und diese Spüllösung verworfen.

Die Trifluormethansulfonsäurelösung wurde wie in Beispiel 71 aufgearbeitet. Man erhielt 11,8 g farblose Kristalle (43,91 mmol, 93,2% d.Th.). HPLC-Analyse der Carbonsäure ergab eine Reinheit von 98,7 FI.-%.

### Beispiel 73

Möglichkeit der Aufarbeitung präparativer Carbonylierungsansätze mit Natronlauge anstatt Natriumcarbonatlösung. Stabilität von 2-[4-(4-Chloro-butyryl)-phenyl]-2-methyl-propionsäure (Formel I, R¹ = R² = Methyl; R³ = 4-Chlorbutyryl) gegen 1 M Natronlauge unter strikter pH-Kontrolle bei pH 10.

Es ist bekannt, dass sich die Chlorketosäure (Formel I, R³ = 4-Chlorbutyryl) bereits unter schwach basischen Bedingungen leicht in die Cyclopropylketosäure (Formel I, R³ = Cyclopropyl) umwandelt. Es wurde untersucht, ob die Chlorketosäure unter strikter pH-Kontrolle bei pH 10 ohne Ringschluß in 1 M Natronlauge extrahiert werden kann, so dass eine Aufarbeitung der Carbonylierungslösung mit 1 M Natronlauge anstatt Natriumcarbonatlösung möglich ist.

2,71 g (10 mmol) 2-[4-(4-Chloro-butyryl)-phenyl]-2-methyl-propionsäure (HPLC-Reinheit 99,4 FI.%, Produkt aus Beispiel 71) wurde in 24 ml Dichlormethan gelöst. 1 ml Wasser wurde zugegeben und dann im Eisbad bei 0 bis +5 °C Innentemperatur mit 10,3 ml 1 M Natronlauge anfangs per Hand, später per Spritzenpumpe, innerhalb 25 Minuten exakt auf pH 10,0 gestellt. Der pH-Wert verharrte dabei lange bei 7,65 und stieg nach Zugabe von einem Äquivalent Natronlauge (10,0 ml) sehr rasch an. In dieser letzten Phase der Titration mußte die Natronlauge tröpfchenweise zugegeben werden, um ein Überschiessen über pH 10,0 zu vermeiden. Die organische Phase wurde abgetrennt. Die wässrige Phase wurde mittels durchperlendem Stickstoff von restlichem Dichlormethan befreit, dann bei 0 bis +5 °C unter Eiskühlung mit 2 ml 30%iger Salzsäure bis pH 1 angesäuert. Die Suspension wurde weitere 30 Minuten im Eisbad nachgerührt, der Feststoff abgesaugt, mit 5 ml Eiswasser gewaschen und im Hochvakuum über Phosphorpentoxid getrocknet. Man erhielt 2,60 g (9,67 mmol, 96,7% der Theorie) farblose Kristalle, die gemäß HPLC-Analyse eine Reinheit von 99,0 FI.% hatten. Die Verunreinigung Cyclopropylketosäure war nur zu 0,3% FI.% enthalten, die Verunreinigung Hydroxyketosäure (Formel I, R³ = 4-Hydroxybutyryl) nur zu 0,2 FI.%.

Aufarbeitung von Carbonylierungslösungen mit 1M Natronlauge ist darum möglich.

### Beispiele 74 - 81

### Synthese von 2-[4-(4-Chloro-butyryl)-phenyl]-2-methyl-propionsäure (Formel I, R¹ = R² = Methyl; R³ = 4-Chlorbutyryl) per Carbonylierung des Bromids (VIII, Y = CI) in alternativen Supersäuren

Die Versuchsprozedur war analog zu den Beispielen 55-70. Die Reaktionszeit betrug 4 Stunden. Aluminiumtrichlorid wurde als Feststoff in die Reaktoren A7 und A8 überführt, dann dass Dichlormethan zugegeben, in Reaktor A8 anschließend zusätzlich 4,5 µl Wasser. Der in den Reaktoren A5 und A6 verwendete kommerzielle Bortrifluorid-Phosphorsäure-Komplex (CAS-Nr.: 13669-76-6) hatte Kp. 147°C und d = 1,840. In Reaktor A6 wurde zusätzlich 4,5 µl Wasser zugesetzt.

| Autoklaven-Nr. | Edukt [mmol] | Säure 0,5 ml | gesamter Wassergehalt [bezogen auf Edukt] | CO-Druck [bar] | Temp. [°C] | Ausbeute (I) [% d. Th.] |
|---|---|---|---|---|---|---|
| A1 | Bromid | CF₃SO₃H | etwa 50 | 40 | 0 | 67 |
| | 0,206 | 98% | mol% | | | |
| A2 | Bromid | FSO₃H 98% | nicht | 40 | 0 | 36 |
| | 0,206 | | bekannt | | | |
| A3 | Bromid | CH₃SO₃H | nicht | 40 | 0 | 1 |
| | 0,206 | | bekannt | | | |
| A4 | Bromid | 40%CH₃SO₃H | nicht | 40 | 0 | 16 |
| | 0,237 | 60% | bekannt | | | |
| | | CF₃SO₃H | | | | |
| A5 | Bromid | BF₃ x H₃PO₄ | nicht | 40 | 0 | 5 |
| | 0,206 | | bekannt | | | |
| A6 | Bromid | BF₃ x H₃PO₄ | 100 mol% | 40 | 0 | 17 |
| | 0,243 | | | | | |
| A7 | Bromid | 10% AlCl₃ | nicht | 40 | 0 | 0 |
| | 0,206 | in CH₂Cl₂ | bekannt | | | |
| A8 | Bromid | 10% AlCl₃ | 100 mol% | 40 | 0 | 3 |
| | 0,243 | in CH₂Cl₂ | | | | |

### Beispiel 82

### Synthese von Cyclopropyl-(4-isopropyl-phenyl)-methanon (Formel III, R¹ = R⁴ = Methyl, R³ = Cyclopropylcarbonyl)

In einem 4L-Vierhalsrundkolben mit Glasrührwelle, PTFE-Rührblatt, Pt 100 Thermometer und pH-Elektrode wurden 457,9 g (2,037 mol) 4-Chlor-1-(4-isopropyl-phenyl)-butan-1-on (Formel III, R¹ = R² = Methyl, R³ = 4-Chlorbutyryl) in 1832 g Methanol bei 40°C unter Rühren gelöst (pH-Wert 0,7). 250 ml Wasser wurden zugegeben (pH-Wert 2,17) und der pH-Wert mit 32%iger wässriger Natronlauge auf 12,6 gestellt. Während der Reaktionszeit von 2,5 Stunden wurde der abfallende pH-Wert immer wieder mit Natronlauge auf pH 12,5 zurückgestellt. Der Gesamtverbrauch Natronlauge betrug 272,0 g. Um das entstehende Natriumchlorid zu lösen wurden während der Reaktion portionsweise 200 ml Wasser zugegeben. Der Rückflusskühler wurde durch eine kurze Claisenbrücke ersetzt und dann im Vakuum (ca. 60 mbar) bei 30°C Methanol / Wasser abdestilliert. Während der Destillation begann Feststoff auszufallen . Um die Suspension rührfähig zu halten wurden nochmals 450 ml Wasser zugegeben. Nachdem die Masse des Destillats 980 g betrug, wurde der Feststoff abgesaugt (feucht 352 g, trocken 325 g), die Mutterlauge auf +2°C abgekühlt und nach 30 Minuten der nochmals ausfallende Feststoff ebenfalls abgesaugt (feucht 35 g, trocken 33 g). Die Masse der Mutterlauge betrug danach 827 g. Gesamtausbeute: 358 g (1,902 mol, 93,3% d.Th.). Die Reinheit dieses Rohprodukts betrug >99,7% FI.-% gemäß 100%-Analyse im GC und HPLC. Im ¹H- und ¹³C-NMR waren keine Verunreinigungen sichtbar. Das Produkt enthielt aber noch geringe anorganische Bestandteile und hatte einen Wassergehalt von 0,89 Gew.-% (Karl-Fischer-Titration), der sich im Vakuum über Phosphorpentoxid nicht in einem vernünftigen Zeitraum auf den für die Folgereaktion angestrebten Wassergehalt von <0,1% verringern ließ. 212,7 g des Rohprodukts wurden in einem 1L-Erlenmeyerkolben in 500 ml n-Heptan bei 20°C trübe gelöst, wobei die auftretende Abkühlung mit einem warmen Wasserbad ausgeglichen wurde. Die Lösung wurde durch eine Klärschicht filtriert und mit n-Heptan nachgewaschen. Das klare, farblose Filtrat wurde im Vakuum eingeengt. Man erhielt ein farbloses Öl, das beim Abkühlen mit Trockeneis im Vakuum fast vollständig durchkristallisierte. Das Restlösungsmittel wurde durch Trocknung im Hochvakuum entfernt. Man erhielt 207,3 g farblose Kristalle (97,4% Rückgewinnung Gew./Gew.), GC: 99,9% FI.-ig, Wassergehalt (Karl-Fischer): 0,06%. ¹H-NMR (CDCl₃): δ = 1,02 (ddd, 2H, 2 x CH), 1,22 (ddd, 2H, 2 x CH), 1,27 (d, 6H, 2 x CH₃), 2,66 (tt,1H, CH), 2,97 (sept., 1H, CH), 7,32 (∼d, 2H, arom.-H), 7,96 (∼d, 2H, arom.-H). ¹³C-NMR (CDCl₃): δ =11,48 (2 x CH₂), 17,09 (CH), 23,84 (2 x CH₃), 34,36 (CH), 126,70 (2 x arom.-CH), 128,39 (2 x arom.-CH), 136,02 (arom.-C), 154,31 (arom.-H), 200,31 (C=O).

### Beispiel 83

### Synthese von [4-(1-Chloro-l-methyl-ethyl)-phenyl]-cyclopropyl-methanon [Formel II, X= CI, R¹ = R⁴ = Methyl, R³ = Cyclopropylcarbonyl) per benzylischer Chlorierung mit tert.-Butylhypochlorit unter Belichtung

In einem 1L-Vierhalskolben mit mechanischem Rührer, Thermometer, Rückflusskühler mit Blasenzähler und Gaseinleitungsrohr für Inertgas wurden 47,5 g (250 mmol) Cyclopropyl-(4-isopropyl-phenyl)-methanon (aus Beispiel 82) in 475 ml Chlorbenzol (Merck Darmstadt) gelöst und die Lösung 15 Min. mit Stickstoff durchperlt um gelösten Sauerstoff auszutreiben. Anschließend wurde mit einem Eis-Kochsalz-Bad in einem verspiegelten Dewargefäß auf -9°C gekühlt und dann die Inertgaszufuhr abgestellt. 43,5 ml (375 mmol, 1,5 Äquiv.) tert.-Butylhypochlorit wurden zugegeben und das Stickstoffeinleitungsrohr durch einen Stopfen ersetzt. Anschließend wurde unter intensivem Rühren mit einer Osram Ultra Vitalux 300 W Lampe ("Sunlamp") bestrahlt. Unter voller Kühlung stieg die Reaktionstemperatur innerhalb der ersten 5 Minuten auf den Maximalwert von 26°C an, wobei sich die anfänglich gelbe Lösung vollständig entfärbte. Innerhalb der nächsten 4 Minuten, weiterhin unter voller Kühlung, fiel die Reaktionstemperatur dann wieder auf +5°C ab und wurde dann, unter schwacher Kühlung, weitere 6 Minuten bei 0 - +1°C gehalten. Die Lampe wurde ausgeschaltet. Nach weiteren 20 Minuten im Kältebad wurde die farblose Lösung im guten Vakuum eingeengt und der ölige Rückstand unter magnetischem Rühren im Hochvakuum getrocknet. Das noch nicht vollständig getrocknete Produkt stand über Nacht unter Schutzgas im Kühlschrank, wobei es kristallisierte. Es wurde weiter im Hochvakuum getrocknet, wobei 57,3 g eines teilkristallinen dicken Breis erhalten wurden. Dieser wurde über eine Glasfritte unter Stickstoffüberlagerung scharf abgesaugt. Die Kristalle wurden mit wenig eiskaltem n-Heptan 2 mal gewaschen und unter Stickstoff trockengesaugt. Erhalten wurden 25,2 g farblose grobe Kristalle, Schmp. 37,5°C. Aus der Mutterlauge kristallisierten nach dem Entfernen des Lösungsmittels im Vakuum und anschließendem Stehen über Nacht im Kühlschrank weitere 12,4 g. Gesamtausbeute: 37,6 g (168,8 mmol, 67,5% d.Th.). Die GC-Reinheit betrug 98,2% FI.-%, der Restgehalt an Edukt 0,5% Fl.-%, der Gehalt an homobenzylischem Chlorid 0,4% Fl.-% und der Gehalt an Dichlorid 0,9% FI.-%. ¹H-NMR (CDCl₃): δ = 1,05 (ddd, 2H, 2 x CH), 1,25 (ddd, 2H, 2 x CH), 2,01 (s, 6H, 2 x CH₃), 2,66 (tt,1H, CH), 7,68 (∼d, 2H, arom.-H), 7,99 (∼d, 2H, arom.-H). ¹³C-NMR (CDCl₃): δ = 11,77 (2 x CH₂), 7,31 (CH), 34,25 (2 x CH₃), 68,95 (C-Cl), 125,81 (2 x arom.-CH), 128,21 (2 x arom.-CH), 137,18 (arom.-C), 150,92 (arom.-C), 200,06 (C=0).

Eine analoge Photoreaktion mit 380 mg (2 mmol) Cyclopropyl-(4-isopropyl-phenyl)-methanon (aus Beispiel 82) in 3,8 ml Chlorbenzol mit 0,35 ml (3 mmol) tert.-Butylhypochlorit ergab das benzylische Chlorid ohne vorherige Reinigung durch Kristallisation mit einer GC-Reinheit von 91 FI.-% in 100%iger Ausbeute (Gew./Gew.).

Eine analoge Photoreaktion mit 380 mg (2 mmol) Cyclopropyl-(4-isopropyl-phenyl)-methanon (aus Beispiel 82) in 3,8 ml Benzotrifluorid (α,α,α-Trifluortoluol) mit 0,35 ml (3 mmol) tert.-Butylhypochlorit ergab das benzylische Chlorid ohne vorherige Reinigung durch Kristallisation mit einer GC-Reinheit von 88 FI.-% in 97%iger Ausbeute (Gew./Gew.).

### Beispiel 84

### Synthese von 1-[4-(1-Chlor-1-methyl-ethyl)-phenyl]-4-chlor-butan-1-on (Formel IX, R¹ = R⁴ = Methyl) per Cyclopropyl-Öffnung mit Chlorwasserstoff

In einem 50 ml Dreihalskolben mit Magnetrührfisch, Thermometer und Rückflusskühler wurden unter Stickstoff 23,7 g (105 mmol) [4-(1-Chloro-1-methyl-ethyl)-phenyl]-cyclopropylmethanon (aus Beispiel 83) bei 38°C aufgeschmolzen und 5 Minuten mit Stickstoff durchperlt, dann unter weiterem Durchperlen von Stickstoff auf 115°C Innentemperatur aufgeheizt. Bei dieser Temperatur wurde Chlorwasserstoffgas aus einer lecture-bottle langsam durchgeperlt. Reaktions-Monitorring per GC zeigte 6% restliches Edukt nach 4 Stunden und 2% restliches Edukt nach 7 Stunden an. Das Reaktionsgemisch wurde unter fortgesetztem einleiten von HCl im Eisbad abgekühlt, dann Stickstoff durchgeperlt um restliches HCl zu verdrängen. Die Wägung der Chlorwasserstoff-lecture-bottle ergab, dass insgesamt 53,2 g (1,47 mol) HCl-Gas eingeleitet worden waren. Das Reaktionsprodukt wurde im Hochvakuum entgast, wobei das Öl wesentlich heller wurde. Erhalten wurden 26,9 g (103,8 mmol, 98,8% d.Th. Gew./Gew.) gelbes Öl, welches nach Tagen im Tiefkühlschrank zu kristallisieren begann und schließlich durchkristallisierte. GC-Analyse des gelben Öls ergab eine Reinheit von 95,0 FI.-%. Das Öl enthielt 1,7 FI.-% unumgesetztes Edukt und 3,3 FI.-% Verunreinigungen. ¹H-NMR (CDCl₃): δ = 2,00 (s, 6H, 2 x CH₃), 2,23 (qui, 2H, CH₂), 3,18 (t, 2H, CH₂), 3,68 (t, 2H, CH₂), 7,68 (∼d, 2H, arom.-H), 7,96 (∼d, 2H, arom.-H).

### Beispiele 85 und 86

### Synthese von 2-[4-(4-Chloro-butyryl)-phenyl]-2-methyl-propionsaure (Formel I, R¹ = R² = Methyl, R³ = 4-Chlorbutyryl) per Carbonylierung des Chlorids (Formel IX, R¹ = R⁴ = Methyl) in Trifluormethan-sulfonsäure mit einem Wassergehalt von ca. 50 mol% bezogen aufs Edukt

Die Carbonylierungen wurden als Doppelansatz parallel in zwei 2 ml Autoklaven durchgeführt, wie in Beispielen 23 und 24 fürs entsprechende Bromid beschrieben. Dabei wurden jeweils 0,206 mmol des Chlorids (aus Beispiel 84) in 0,5 ml 98%iger Trifluormethansulfonsäure (Wassergehalt ca. 50 mol% bezogen aufs Edukt) unter 40 bar CO-Druck bei 0°C für 4 Stunden reagieren gelassen. HPLC-Analyse ergab nach der Methode des externen Standards (beschrieben in Beispiel 3 - 10) eine Ausbeute der gewünschten Carbonsäure von 67% bzw. 69% d.Th.. Ausbeute und Zusammensetzung der rohen Carbonylierungslösung waren vergleichbar dem Ergebnis, das unter gleichen Reaktionsbedingungen mit dem Bromid als Edukt erzielt worden war (vergleiche Beispiel 74).

### Beispiele 87 und 88

### Synthese von 2-[4-(4-Chloro-butyryl)-phenyl]-2-methyl-propionsäure (Formel I, R¹ = R² = Methyl, R³ = 4-Chlorbutyryl) per Carbonylierung des Chlorids (Formel IX, R¹ = R⁴ = Methyl) in Fluorsulfonsäure

Die Carbonylierungen wurden als Doppelansatz parallel in zwei 2 ml Autoklaven durchgeführt wie in Beispielen 23 und 24 fürs entsprechende Bromid beschrieben. Dabei wurden jeweils 0,206 mmol des Chlorids (aus Beispiel 84) in 0,5 ml 98%iger Fluorsulfonsäure (Wassergehalt nicht ermittelt) unter 40 bar CO-Druck bei 0°C für 4 Stunden reagieren gelassen. HPLC-Analyse ergab nach der Methode des externen Standards (beschrieben in Beispiel 3 - 10) eine Ausbeute der gewünschten Carbonsäure von 29% bzw. 31% d.Th.. Ausbeute und Zusammensetzung der rohen Carbonylierungslösung waren vergleichbar dem Ergebnis, das unter gleichen Reaktionsbedingungen mit dem Bromid als Edukt erzielt worden war (vergleiche Beispiel 75).

### Beispiele 89 und 90

### Synthese von 2-[4-(4-Chloro-butyryl)-phenyl]-2-methyl-propionsäure (Formel I, R¹ = R² = Methyl, R³ = 4-Chlorbutyryl)per Carbonylierung des Chlorids (Formel IX, R¹ = R⁴ = Methyl) in Trifluormethan-sulfonsäure mit einem Wassergehalt von ca. 200 mol% bezogen aufs Edukt

Die Carbonylierungen wurden als Doppelansatz parallel in zwei 2 ml Autoklaven durchgeführt wie in Beispielen 23 und 24 fürs entsprechende Bromid beschrieben. Dabei wurden jeweils 0,206 mmol des Chlorids (aus Beispiel 84) in 0,5 ml 98%iger Trifluormethansulfonsäure (Wassergehalt ca. 200 mol% bezogen aufs Edukt) unter 40 bar CO-Druck bei 0°C für 20 Stunden reagieren gelassen. HPLC-Analyse ergab nach der Methode des externen Standards (beschrieben in Beispiel 3 - 10) eine Ausbeute der gewünschten Carbonsäure von 94% bzw. 95% d. Th.. Ausbeute und Zusammensetzung der rohen Carbonylierungslösung waren vergleichbar mit dem Ergebnis, das unter gleichen Reaktionsbedingungen mit dem Bromid als Edukt erzielt worden war (vergleiche Beispiel 68).

## Patentansprüche

1. Verfahren zur Gewinnung der Verbindung der Formel I 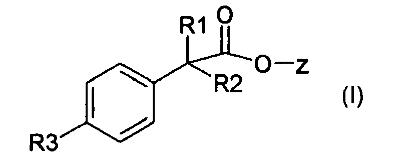 und/oder ein Salz der Verbindung der Formel I, wobei
R1 und R2 gleich oder verschieden sind und unabhängig voneinander für -(C₁-C₄)-Alkyl stehen,
R3 für 1) -C(O)-(C₁-C₄)-Alkyl, worin Alkyl einfach durch Cl oder Br substituiert ist, oder 2) -C(O)-(C₃-C₆)-Cycloalkyl, steht und
Z für Wasserstoffatom oder -(C₁-C₁₀)-Alkyl steht,
das **dadurch gekennzeichnet ist, dass** man eine Verbindung der Formel II, 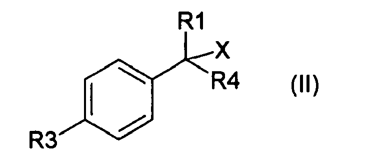 wobei
R1 und R3 die selben Bedeutungen wie in Formel I haben,
X für Cl, Br oder -OH steht und
R4 die selbe Bedeutung wie der Rest R2 in Formel I hat oder zusammen mit X eine C=C-Doppelbindung darstellt,
mit Kohlenmonoxid oder einer kohlenmonoxid-freisetzenden Verbindung in Gegenwart von konzentrierter Schwefelsäure, Hydrogenfluorid oder einer Supersäure oder Mischungen derselben umsetzt und anschließend
a) Wasser zufügt, um die Verbindung der Formel I zu erhalten, worin Z für Wasserstoffatom steht oder
b) (C₁-C₁₀)-Alkyl-OH zufügt, wenn X für Cl oder Br steht oder R4 zusammen mit X eine C=C-Doppelbindung darstellt, um die Verbindung der Formel I zu erhalten, worin Z für -(C₁-C₁₀)-Alkyl steht.

2. Verfahren gemäß Anspruch 1, **dadurch gekennzeichnet, dass** man eine Verbindung der Formel I gewinnt, wobei
R1 und R2 gleichzeitig für Methyl stehen,
R3 für 1. -C(O)-Propyl, worin Propyl einfach durch Cl substituiert ist, oder 2. -C(O)-Cyclopropyl, steht und
Z für Wasserstoffatom oder -(C₁-C₄)-Alkyl steht.

3. Verfahren gemäß Anspruch 1 oder 2, **dadurch gekennzeichnet, dass** man als kohlenmonoxid-freisetzende Verbindung mindestens eine Verbindung aus der Gruppe Ameisensäure, Ameisensäuresalze mit anorganischen oder organischen Kationen oder Metallcarbonyle einsetzt.

4. Verfahren gemäß einem oder mehreren der Ansprüche 1 bis 3, **dadurch gekennzeichnet, dass** man als Supersäure eine Säure aus der Gruppe Perchlorsäure, Chlorsulfonsäure, Fluorsulfonsäure, Trifluormethansulfonsäure, Perfluorbutan-1-sutfonsäure oder Lewis-Säuren wie SO₃, Aluminiumtrichlorid oder Antimonpentafluorid oder konjugierte Protonensäure-Lewissäure-Komplexe wie Schwefelsäure mit SO₃, Schwefelsäure mit Borsäure, Fluorsulfonsäure mit Antimonpentafluorid, Trifluormethansulfonsäure mit Antimonpentafluorid, Hydrogenfluorid mit Antimonpentafluorid, HF mit TaF₅, BF₃ mit HF (HBF₄, Tetrafluoroborsäure), BF₃ x H₃PO₄ - Komplex, oder Fluorsulfonsäure mit SO₃, oder konjugierte Komplexe von HSO₃F mit HF und SbF₅, oder HSO₃F mit SO₃ und SbF₅ einsetzt.

5. Verfahren gemäß Anspruch 4, **dadurch gekennzeichnet, dass** man eine immobilisierte Supersäure einsetzt, insbesondere immobilisierte Trifluormethansulfonsäure oder Fluorsulfonsäure.

6. Verfahren gemäß einem oder mehreren der Ansprüche 1 bis 5, **dadurch gekennzeichnet, dass** man die Umsetzung in Gegenwart von Lösungsmitteln durchgeführt, die gegenüber Supersäuren ausreichend inert sind, aus der Gruppe flüssiges Schwefeldioxid, superkritisches Kohlendioxid, Sulfolan, n-Alkane mit 4 bis 12 Kohlenstoffatomen, Chlorbenzol, Fluorbenzol, Toluol, Cumol, halogenierte Kohlenwasserstoffe, Methylacetat, Ethylacetat oder n-Butylacetat, durchführt.

7. Verfahren gemäß einem oder mehreren der Ansprüche 1 bis 6, **dadurch gekennzeichnet, dass** man die Umsetzung in Gegenwart eines Additivs durchführt, das unter Kohlenmonoxid-Kontakt rasch in Metallcarbonyle übergeht wie Kupfer(I)-oxid, Silber(I)-oxid, Silbernitrat, Eisenpentacarbonyl Fe(CO)₅, Dinatriumtetracarbonylferrat(-2) Na₂Fe(CO)₄, Octacarbonyldicobalt(O) Co₂(CO)₈ oder Nickeltetracarbonyl Ni(CO)₄.

8. Verfahren gemäß einem oder mehreren der Ansprüche 1 bis 7, **dadurch gekennzeichnet, dass** man die Verbindung der Formel I, worin Z für -(C₁-C₁₀)-Alkyl steht, in den entsprechenden Alkohol und Carbonsäure der Formel I (Z=H) spaltet.

9. Verfahren gemäß einem oder mehreren der Ansprüche 1 bis 8, **dadurch gekennzeichnet, dass** man die Umsetzung bei einem CO-Druck von 1 bar bis 500 bar, bevorzugt von 1 bis 40 bar, besonders bevorzugt von 5 bis 25 bar, durchführt und bei einer Reaktionstemperatur von -70 °C bis +100 °C, bevorzugt von -10 °C bis +50°C, besonders bevorzugt von 0 °C bis +40 °C durchführt.

10. Verfahren gemäß einem oder mehreren der Ansprüche 1 bis 9, **dadurch gekennzeichnet, dass** man pro Liter Supersäure von 0,1 Mol bis 5,0 Mol der Verbindung der Formel II eingesetzt, insbesondere von 0,3 Mol bis 3,0 Mol, bevorzugt von 0,4 Mol bis 2,0 Mol.

11. Verfahren gemäß einem oder mehreren der Ansprüche 1 bis 10, **dadurch gekennzeichnet, dass** man das Additiv in einer Menge von 5 Mol-% bis 100 Mol-% bezogen auf die Verbindung der Formel II eingesetzt, bevorzugt von 10 bis 30 Mol-%, insbesondere etwa 20 Mol-%.

12. Verfahren gemäß einem oder mehreren der Ansprüche 1 bis 11, **dadurch gekennzeichnet, dass** man die Verbindung der Formel II mit Kohlenmonoxid oder einer kohlenmonoxid-freisetzenden Verbindung in Gegenwart von Wasser, wobei Wasser in einer Menge von 2 Mol-% bis 800 Mol-% bezogen auf die Verbindung der Formel II vorliegt, und einer konzentrierten Schwefelsäure oder Hydrogenfluorid oder einer Supersäure oder Mischungen derselben, umsetzt.

13. Verfahren gemäß Anspruch 12, **dadurch gekennzeichnet, dass** eine Verbindung der Formel II, wobei X für Brom, R1 für Methyl, R2 für Methyl und R3 für 4-Chlorbutyryl steht, mit Kohlenmonoxid in Gegenwart von Trifluormethansulfonsäure und Wasser, wobei Wasser in einer Menge von 50 Mol-% bis 500 Mol-% bezogen auf die Verbindung der Formel II vorliegt, umgesetzt wird.

14. Verfahren gemäß Anspruch 13, **dadurch gekennzeichnet, dass** Wasser in einer Menge von 90 Mol-% bis 300 Mol-%, insbesondere etwa 200 Mol-%, bezogen auf die Verbindung der Formel II eingesetzt wird.

15. Verbindung der Formel X, 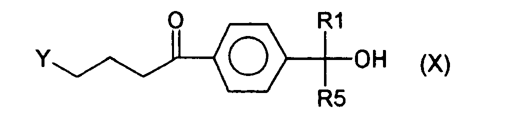
wobei Y Chlor- oder Bromatom, bedeutet, und
R1 und R5 gleich oder verschieden sind und unabhängig voneinander für -(C₁-C₄)-Alkyl stehen.

16. Verbindung der Formel X gemäß Anspruch 15, worin
Y ein Chloratom bedeutet und R1 und R5 jeweils für Methyl stehen.

17. Verfahren zur Gewinnung von Verbindungen der Formel X gemäß Anspruch 15, **dadurch gekennzeichnet, dass** man
a) eine Verbindung der Formel III, 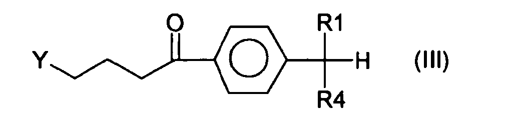 wobei Y Chlor- oder Bromatom, bedeutet, und R1 und R4 gleich oder verschieden sind und unabhängig voneinander für -(C₁-C₄)-Alkyl stehen, mit Sauerstoff in Gegenwart von Kobalt(II)acetat-Tetrahydrat und N-Hydroxyphthalimid umsetzt, oder
b) eine Verbindung der Formel XI, 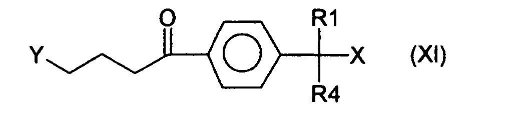
wobei Y Chlor- oder Bromatom, bedeutet,
X für Chlor- oder Bromatom steht,
R1 und R4 gleich oder verschieden sind und unabhängig voneinander für - (C₁-C₄)-Alkyl stehen oder
R4 und X zusammen eine C=C-Doppelbindung darstellen,
mit Wasser umsetzt.

## Claims

1. A process for obtaining the compound of the formula I 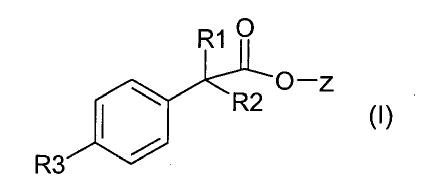 and/or a salt of the compound of the formula I where
R1 and R2 are the same or different and are each independently -(C₁-C₄)-alkyl,
R3 is
1) -C(O)-(C₁-C₄)-alkyl in which alkyl is monosubstituted by Cl or Br, or
2) -C(O)-(C₃-C₆)-cycloalkyl, and
Z is a hydrogen atom or -(C₁-C₁₀)-alkyl,
which comprises reacting a compound of the formula II 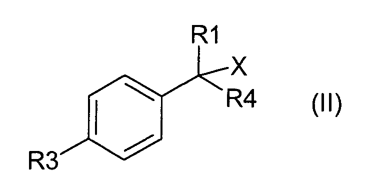 where
R1 and R3 are each as defined in formula I,
X is CI, Br or -OH and
R4 is as defined for the R2 radical in formula I or, together with X, is a C=C double bond
with carbon monoxide or a carbon monoxide-releasing compound in the presence of concentrated sulfuric acid, hydrogen fluoride or of a superacid or mixtures thereof, and then
a) adding water in order to obtain the compound of the formula I in which Z is a hydrogen atom or
b) adding (C₁-C₁₀)-alkyl-OH when X is Cl or Br, or R4, together with X, is a C=C double bond, in order to obtain the compound of the formula I in which Z is -(C₁-C₁₀)-alkyl.

2. The process as claimed in claim 1, wherein a compound of the formula I is obtained where
R1 and R2 are simultaneously methyl,
R3 is 1. -C(O)-propyl where propyl is monosubstituted by Cl or 2. -C(O)-cyclopropyl and
Z is a hydrogen atom or -(C₁-C₄)-alkyl.

3. The process as claimed in claim 1 or 2, wherein the carbon monoxide-releasing compound used is at least one compound from the group of formic acid, formate salts with inorganic or organic cations or metal carbonyls.

4. The process as claimed in one or more of claims 1 to 3, wherein the superacid used is an acid from the group of perchloric acid, chlorosulfonic acid, fluorosulfonic acid, trifluoromethanesulfonic acid, perfluorobutane-1-sulfonic acid, or Lewis acids such as SO₃, aluminum trichloride or antimony pentafluoride or conjugated protic acid-Lewis acid complexes such as sulfuric acid with SO₃, sulfuric acid with boric acid, fluorosulfonic acid with antimony pentafluoride, trifluoromethanesulfonic acid with antimony pentafluoride, hydrogen fluoride with antimony pentafluoride, HF with TaF₅, BF₃ with HF (HBF₄, tetrafluoroboric acid), BF₃ . H₃PO₄ complex, or fluorosulfonic acid with SO₃, or conjugated complexes of HSO₃F with HF and SbF₅, or HSO₃F with SO₃ and SbF₅.

5. The process as claimed in claim 4, wherein an immobilized superacid is used, in particular immobilized trifluoromethanesulfonic acid or fluorosulfonic acid.

6. The process as claimed in one or more of claims 1 to 5, wherein the reaction is carried out in the presence of solvents which are sufficiently inert toward superacids, from the group of liquid sulfur dioxide, supercritical carbon dioxide, sulfolane, n-alkanes having from 4 to 12 carbon atoms, chlorobenzene, fluorobenzene, toluene, cumene, halogenated hydrocarbons, methyl acetate, ethyl acetate or n-butyl acetate.

7. The process as claimed in one or more of claims 1 to 6, wherein the reaction is carried out in the presence of an additive which is converted rapidly under contact with carbon monoxide to metal carbonyls, such as copper(I) oxide, silver(I) oxide, silver nitrate, iron pentacarbonyl Fe(CO)₅, disodium tetracarbonylferrate(-2) Na₂Fe(CO)₄, octacarbonyldicobalt(0) Co₂(CO)₈ or nickel tetracarbonyl Ni(CO)₄.

8. The process as claimed in one or more of claims 1 to 7, wherein the compound of the formula I in which Z is -(C₁-C₁₀)-alkyl is cleaved to the corresponding alcohol and carboxylic acid of the formula I (Z=H).

9. The process as claimed in one or more of claims 1 to 8, wherein the reaction is carried out at a CO pressure of from 1 bar to 500 bar, preferably from 1 to 40 bar, more preferably from 5 to 25 bar and carried out at a reaction temperature of from -70°C to +100°C, preferably from -10°C to +50°C, more preferably from 0°C to +40°C.

10. The process as claimed in one or more of claims 1 to 9, wherein from 0.1 mol to 5.0 mol of the compound of the formula II are used per liter of superacid, in particular from 0.3 mol to 3.0 mol, preferably from 0.4 mol to 2.0 mol.

11. The process as claimed in one or more of claims 1 to 10, wherein the additive is used in an amount of from 5 mol% to 100 mol% based on the compound of the formula II, preferably from 10 to 30 mol%, in particular about 20 mol%.

12. The process as claimed in one or more of claims 1 to 11, wherein the compound of the formula II is reacted with carbon monoxide or a
carbon monoxide-releasing compound in the presence of water, water being present in an amount of from 2 mol% to 800 mol% based on the compound of the formula II, and of concentrated sulfuric acid or hydrogen fluoride or of a superacid or mixtures thereof.

13. The process as claimed in claim 12, wherein a compound of the formula II where X is bromine, R1 is methyl, R2 is methyl and R3 is 4-chlorobutyryl is reacted with carbon monoxide in the presence of trifluoromethanesulfonic acid and water, water being present in an amount of from 50 mol% to 500 mol% based on the compound of the formula II.

14. The process as claimed in claim 13, wherein water is used in an amount of from 90 mol% to 300 mol%, in particular about 200 mol%, based on the compound of the formula II.

15. A compound of the formula X 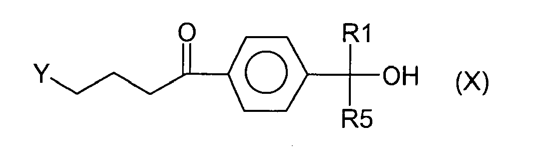
where Y is a chlorine or bromine atom, and
R1 and R5 are the same or different and are each independently -(C₁-C₄)-alkyl.

16. A compound of the formula X as claimed in claim 15, in which Y is a chlorine atom and R1 and R5 are each methyl.

17. A process for obtaining compounds of the formula X as claimed in claim 15, which comprises
a) reacting a compound of the formula III 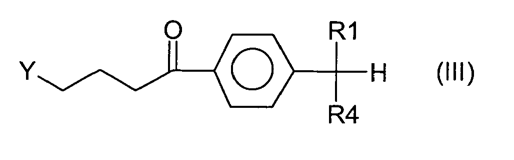 where Y is a chlorine or bromine atom and R1 and R4 are the same
or different and are each independently -(C₁-C₄)-alkyl
with oxygen in the presence of cobalt(II) acetate tetrahydrate and N-hydroxyphthalimide or
b) reacting a compound of the formula XI 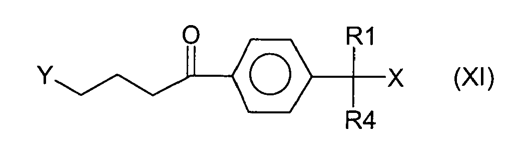
where Y is a chlorine or bromine atom,
X is a chlorine or bromine atom,
R1 and R4 are the same or different and are each independently -(C₁-C₄)-alkyl or
R4 and X together are a C=C double bond
with water.

## Revendications

1. Procédé pour la production du composé de formule I, 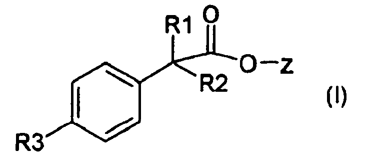 et/ou d'un sel du composé de formule I, où
R1 et R2 sont identiques ou différents et représentent, indépendamment l'un de l'autre, -(C₁-C₄) -alkyle,
R3 représente
1) -C(O)-(C₁-C₄)-alkyle, où alkyle est monosubstitué par C1 ou Br, ou
2) -C(O)-(C₃-C₆)-cycloalkyle, et
Z représente un atome d'hydrogène ou -(C₁-C₁₀)-alkyle,
qui est **caractérisé en ce qu'**on transforme un composé de formule II 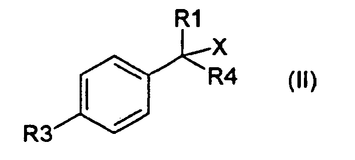 où
R1 et R3 ont les mêmes significations que dans la formule I,
X représente C1, Br ou -OH et
R4 a la même signification que le radical R2 dans la formule I ou représente, ensemble avec X, une double liaison C=C,
avec du monoxyde de carbone ou un composé libérant du monoxyde de carbone en présence d'acide sulfurique concentré, de fluorure d'hydrogène ou d'un superacide ou des mélanges de ceux-ci, puis
a) on ajoute de l'eau, pour obtenir le composé de formule I, où Z représente un atome d'hydrogène ou
b) on ajoute (C₁-C₁₀)-alkyl-OH, lorsque X représente C1 ou Br ou R4 représente, ensemble avec X une double liaison C=C, pour obtenir le composé de formule I, où Z représente -(C₁-C₁₀)-alkyle.

2. Procédé selon la revendication 1, **caractérisé en ce qu'**on produit un composé de formule I, où
R1 et R2 représentent simultanément méthyle,
R3 représente
1. -C(O)-propyle, où propyle est monosubstitué par Cl, ou
2. -C(O)-cyclopropyle, et
Z représente un atome d'hydrogène ou -(C₁-C₄)-alkyle.

3. Procédé selon la revendication 1 ou 2, **caractérisé en ce qu'**on utilise comme composé libérant du monoxyde de carbone au moins un composé du groupe formé par l'acide formique, les sels de l'acide formique avec des cations inorganiques ou organiques ou un métalcarbonyle.

4. Procédé selon l'une ou plusieurs des revendications 1 à 3, **caractérisé en ce qu'**on utilise comme superacide un acide du groupe formé par l'acide perchlorique, l'acide chlorosulfonique, l'acide fluorosulfonique, l'acide trifluorométhanesulfonique, l'acide perfluorobutane-1-sulfonique ou des acides de Lewis tels que SO₃, le trichlorure d'aluminium ou le pentafluorure d'antimoine ou des complexes conjugués d'acide protonique-acide de Lewis, tels que l'acide sulfurique avec SO₃, l'acide sulfurique avec de l'acide borique, l'acide fluorosulfonique avec du pentafluorure d'antimoine, l'acide trifluorométhanesulfonique avec du pentafluorure d'antimoine, le fluorure d'hydrogène avec du pentafluorure d'antimoine, HF avec TaF₅, BF₃ avec HF (HBF₄, acide tétrafluoroborique), le complexe BF₃ x H₃PO₄, ou l'acide fluorosulfonique avec SO₃, ou des complexes conjugués de HSO₃F avec HF et SbF₅, ou de HSO₃F avec SO₃ et SbF₅.

5. Procédé selon la revendication 4, **caractérisé en ce qu'**on utilise un superacide immobilisé, en particulier de l'acide trifluorométhanesulfonique immobilisé ou de l'acide fluorosulfonique immobilisé.

6. Procédé selon l'une ou plusieurs des revendications 1 à 5, **caractérisé en ce qu'**on réalise la transformation en présence de solvants qui sont suffisamment inertes par rapport aux superacides, du groupe formé par le dioxyde de soufre liquide, le dioxyde de carbone supercritique, le sulfolane, les n-alcanes comprenant 4 à 12 atomes de carbone, le chlorobenzène, le fluorobenzène, le toluène, le cumène, les hydrocarbures halogénés, l'acétate de méthyle ou l'acétate de n-butyle.

7. Procédé selon l'une ou plusieurs des revendications 1 à 6, **caractérisé en ce qu'**on réalise la transformation en présence d'un additif qui se transforme rapidement au contact avec du monoxyde de carbone en métalcarbonyle tel que l'oxyde de cuivre (I), l'oxyde d'argent (I), le nitrate d'argent, le fer-pentacarbonyle Fe(CO)₅, le tétracarbonylferrate(-2) disodique Na₂Fe(CO)₄, l'octacarbonyldicobalt (0) Co₂(CO)₈ ou le nickel-tétracarbonyle Ni(CO)₄.

8. Procédé selon l'une ou plusieurs des revendications 1 à 7, **caractérisé en ce qu'**on dissocie le composé de formule I, dans lequel Z représente -(C₁-C₁₀)-alkyle, en alcool correspondant et acide carboxylique de formule I (Z=H).

9. Procédé selon l'une ou plusieurs des revendications 1 à 8, **caractérisé en ce qu'**on réalise la transformation à une pression en CO de 1 bar à 500 bars, de préférence de 1 à 40 bars, de manière particulièrement préférée de 5 à 25 bars, et à une température de réaction de -70°C à +100°C, de préférence de -10°C à +50°C, de manière particulièrement préférée de 0°C à +40°C.

10. Procédé selon l'une ou plusieurs des revendications 1 à 9, **caractérisé en ce qu'**on utilise par litre de superacide 0,1 mole à 5,0 moles du composé de formule II, en particulier 0,3 mole à 3,0 moles, de préférence 0,4 mole à 2,0 moles.

11. Procédé selon l'une ou plusieurs des revendications 1 à 10, **caractérisé en ce qu'**on utilise l'additif en une quantité de 5% en mole à 100% en mole par rapport au composé de formule II, de préférence de 10 à 30% en mole, en particulier d'environ 20% en mole.

12. Procédé selon l'une ou plusieurs des revendications 1 à 11, **caractérisé en ce qu'**on transforme le composé de formule II avec du monoxyde de carbone ou un composé libérant du monoxyde de carbone en présence d'eau, l'eau étant présente en une quantité de 2% en mole à 800% en mole par rapport au composé de formule II, et d'un acide sulfurique concentré ou de fluorure d'hydrogène ou d'un superacide ou des mélanges de ceux-ci.

13. Procédé selon la revendication 12, **caractérisé en ce qu'**on transforme un composé de formule II, où X représente brome, R1 représente méthyle, R2 représente méthyle et R3 représente 4-chlorobutyryle, avec du monoxyde de carbone en présence d'acide trifluorométhanesulfonique et d'eau, l'eau étant présente en une quantité de 50% en mole à 500% en mole par rapport au composé de formule II.

14. Procédé selon la revendication 13, **caractérisé en ce** l'eau est utilisée en une quantité de 90% en mole à 300% en mole, en particulier d'environ 200% en mole, par rapport au composé de formule II.

15. Composé de formule X, 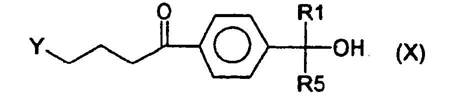 où Y signifie un atome de chlore ou de brome, et
R1 et R5 sont identiques ou différents et représentent, indépendamment l'un de l'autre, -(C₁-C₄)-alkyle.

16. Composé de formule X selon la revendication 15, où
Y signifie un atome de chlore et R1 et R5 représentent à chaque fois méthyle.

17. Procédé pour la production de composés de formule X selon la revendication 15, **caractérisé en ce qu'**on transforme
a) un composé de formule III, 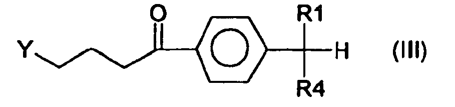 où Y signifie un atome de chlore ou de brome, et R1 et R4 sont identiques ou différents et représentent, indépendamment l'un de l'autre, -(C₁-C₄)-alkyle, avec de l'oxygène en présence d'acétate de cobalt (II) tétrahydraté et du N-hydroxyphtalimide, ou
b) un composé de formule XI, 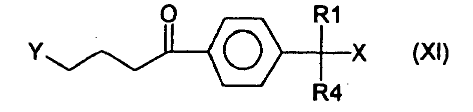
où Y signifie un atome de chlore ou de brome,
X représente un atome de chlore ou de brome,
R1 et R4 sont identiques ou différents et représentent, indépendamment l'un de l'autre, -(C₁-C₄)-alkyle ou
R4 et X représentent ensemble une double liaison C=C,
avec de l'eau.
